(19) Europäisches Patentamt
European Patent Office
Office européen des brevets

(11) Numéro de publication : **0 485 290 A1**

(12) **DEMANDE DE BREVET EUROPEEN**

(21) Numéro de dépôt : **91402980.6**

(22) Date de dépôt : **06.11.91**

(51) Int. Cl.[5] : **C07D 333/38,** C07D 213/80, C10M 135/34, C10M 133/40

(30) Priorité : **07.11.90 FR 9013825**

(43) Date de publication de la demande : **13.05.92 Bulletin 92/20**

(84) Etats contractants désignés : **DE FR GB IT NL**

(71) Demandeur : **NYCO S.A.**
**66, avenue des Champs-Elysées, 51, rue de Ponthieu**
**F-75008 Paris (FR)**

(72) Inventeur : **Leseche, Bernard**
**Société NYCO, 1, rue L.M. Doitteau**
**F-78700 Conflans Sainte Honorine (FR)**

(74) Mandataire : **Harlé, Robert et al**
**c/o Cabinet Harlé & Phélip 21, rue de la Rochefoucauld**
**F-75009 Paris (FR)**

(54) **Nouveaux agents anti-oxydants et/ou anti-corrosion destinés notamment à être utilisés comme additifs dans des compositionslubrifiantes, en particulier pour turbines.**

(57) L'invention est relative à un agent anti-oxydant et/ou anti-corrosion pour une composition lubrifiante ayant la formule

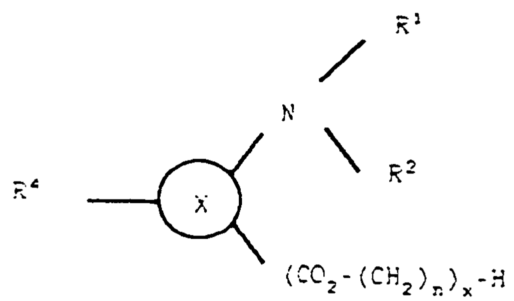

(I)

Eventuellement $R^1$ et $R^2$ ensemble peuvent constituer

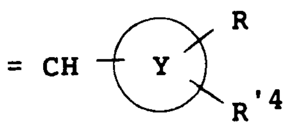

(II)

EP 0 485 290 A1

L'invention est relative à de nouveaux agents anti-oxydants et/ou anti-corrosion destinés notamment à être utilisés comme additifs anti-oxydants et/ou anti-corrosion dans des compositions lubrifiantes à base synthétique ou minérale, telles qu'huiles de graissage, graisses, fluides hydrauliques, et plus particulièrement dans des huiles lubrifiantes destinées à être utilisées à des températures élevées, comme les huiles pour turbine à base ester.

Les nouveaux agents anti-oxydants et/ou anti-corrosion selon l'invention sont ajoutés à titre d'additifs dans les compositions lubrifiantes dans des proportions comprises entre 0,l% et 10%, de préférence entre l% et 3%.

Ces nouveaux agents sont définis par la formule générale suivante:

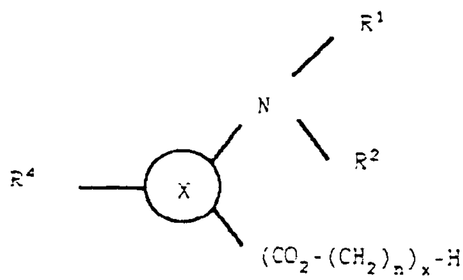

(I)

dans laquelle:

l°) - Ⓧ (parfois désigné ci-après simplement X) représente un cycle aromatique comportant, ou bien un ensemble de deux ou trois cycles aromatiques comportant chacun:

- 5 ou 6 atomes, dont l à 3 ou l à 4 hétéroatomes, en particulier:
- l à 3 hétéroatomes sur un cycle à 5 atomes ou
- l à 4 hétéroatomes sur un cycle à 6 atomes;

2°) - $R^1$ représente un atome d'hydrogène, ou éventuellement un radical alcoyle inférieur, notamment en $C_6$ à $C_{16}$;

3°) - $R^2$ représente:

a. - un atome d'hydrogène,
b. - un alcoyle en $C_6$-$C_{16}$
c. - un groupe ester $CO_2R^5$, $R^5$ désignant un alcoyle en $C_6$-$C_{16}$,
d. - un groupe $(CH_2)_n$-$CO_2R^6$, $R^6$ désignant un alcoyle en $C_6$ à $C_{16}$ et n représentent un nombre entier entre l et 6,
e. - Ar-$R^7$, Ar désignant un radical aryl et $R^7$ un alcoyle en $C_6$ à $C_{16}$ ou $CO_2R^5$, ou
f - un hétérocycle - $R^7$;

4°) - $R^4$ représente:

| | |
|---|---|
| a. - un atome d'hydrogène<br>b. - un alcoyl en $C_6$ à $C_{16}$, ou<br>c. - un phényl | lié:<br>soit à un atome de C<br>soit à un hétéroatome (N); |

5°) - x = O ou l,

- n = un nombre entier de l à 16,

étant entendu que

- si $R^2$ possède un groupement ester (cas c, d, e, f), x = O
- si $R^2$ ne contient pas de groupement ester (cas a, b), x = l.

En variante l'ensemble $R^1$ et $R^2$ peut être constitué par

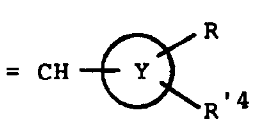

(II)

ce qui correspond à un additif de formule

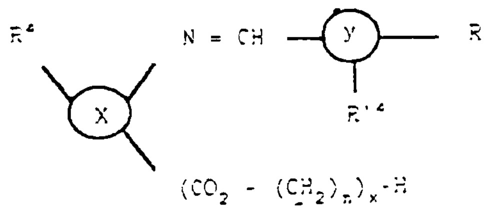

(III)

Dans les formules (II) et III:

6°) Ⓨ (parfois désigné ci-après simplement Y) représente un cycle aromatique (ou sens de Hückel), soit sans hétéroatome (noyau benzénique), soit avec au moins un hétéroatome (N, S ou 0):

7°) - $R'^4$ a la même définition que $R^4$;

8°) - R désigne:

a.- un atome d'hydrogène,

b.- un groupe alcoyle en $C_6$-$C_{16}$,

c.- un groupe ester en $C_6$-$C_{16}$,

étant entendu que

– si R contient un groupement ester (cas c), x = O,

– si R ne contient pas de groupement ester (cas a, b), x = l.

Dans les formules (I), (II) et (III), les différents groupes alcoyle peuvent être ramifiés (sur l'un ou plusieurs des atomes de carbone) ou non.

En ce qui concerne l'application des composés de formule(I) ou (III) pour constituer des additifs anti-oxydants et/ou anti-corrosion pour les huiles de graissage destinées à être utilisées à température élevée, notamment pour les huiles pour turbines, le problème résolu par une telle application est celui de réaliser des huiles de graissage ou lubrifiants, dopées par un additif anti-oxydant et/ou anti-corrosion, ayant une stabilité thermo-oxydante élevée à haute température.

En effet les turbines, notamment pour la propulsion des avions et des hélicoptères, atteignent des températures de plus en plus élevées.

Les huiles de graissage ou lubrifiant pour les turboréacteurs d'avion ou d'hélicoptères sont en général constituées par un ou plusieurs esters avec un ou plusieurs additifs.

Les additifs anti-oxydants et/ou anti-corrosion selon les formules (I) et (III), lorsqu'ils sont ajoutés à une huile de graissage de type ester, permettent à celle-ci de fonctionner correctement à des températures allant jusqu'à 220°C, tout en lui assurant une viscosité de l'ordre de $4.10^{-6}$ $m^2/s$ à 100°C.

On va décrire maintenant l'invention plus en détail, notamment en ce qui concerne les composés des formules (I) et (III) et leurs procédés de préparation, d'une part, et leur application en tant qu'additifs anti-oxydants et/ou anticorrosion d'autre part, étant entendu que la description détaillée qui va suivre n'est donnée qu'à titre illustratif, sans aucun caractère limitatif. Lorsque x=1 dans les formules

A - EXEMPLES DE COMPOSES SELON L'INVENTION

I - Exemples d'esters d'acides hétérocycliques aminés

a) à un seul hétérocycle aromatique

1° - avec un seul hétéroatome

- l'hétéroatome est celui du thiophène

EXEMPLE GENERAL 1

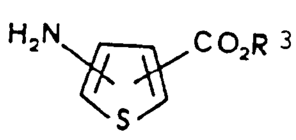

(1)

EXEMPLE ENCORE PLUS GENERAL 2

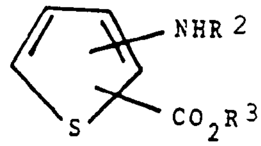

(2)

EXEMPLE LE PLUS GENERAL 3

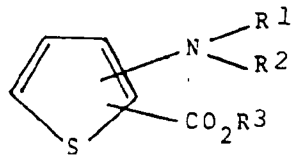

(3)

- l'hétérocycle est celui de la pyridine

EXEMPLE 4

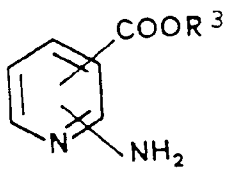

(4)

- l'hétérocycle est celui du pyrrole

EXEMPLE 5

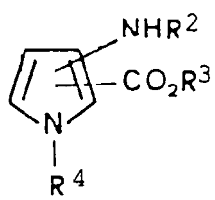

(5)

- l'hétérocycle est celui du furanne

- EXEMPLE 6

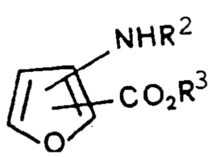

(6)

2° - avec deux hétéroatomes

- l'hétérocycle est celui du pyrazole

EXEMPLE 7

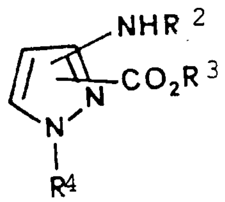

(7)

- l'hétérocycle est celui de l'imidazole

EXEMPLE 8

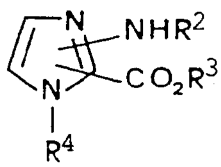

(8)

- l'hétérocycle est celui des oxazoles l,2 ou l,3

EXEMPLE 9

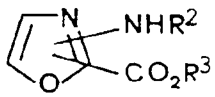

(9)

- l'hétérocycle est celui des thiazoles l,1 ou l,3

EXEMPLE 10

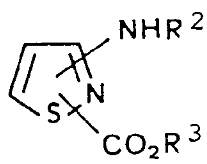

(10)

- l'hétérocycle est celui de la pyrimidine

EXEMPLE 11

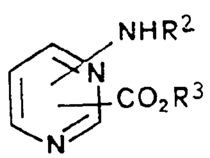

(11)

- l'hétérocycle est celui de la pyrazine

EXEMPLE 12

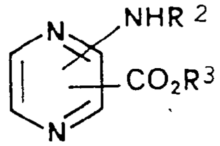

(12)

- l'hétérocycle est celui de la pyridazine

EXEMPLE 13

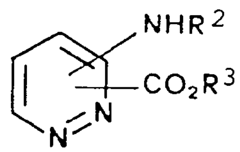

(13)

3° - avec 3 hétéroatomes

- le cycle est celui des thiadiazoles, en particulier de la l,2,5-thiadiazole

EXEMPLE 14

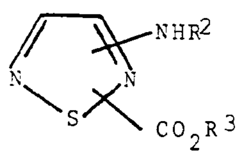

(14)

- l'hétérocycle est celui du triazole

EXEMPLE 15

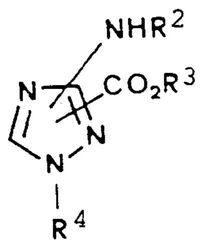

(15)

- l'hétérocycle est celui des triazines l,2,3 ou l,2,4 ou l,3,5, soit respectivement:

EXEMPLE 16

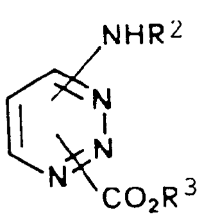

(16)

EXEMPLE 17

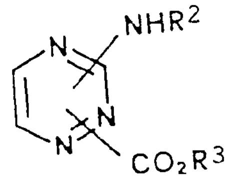

(17)

EXEMPLE 18

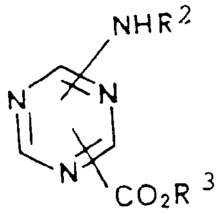

(18)

b) - à deux cycles aromatiques, dont au moins l'un possède un hétéroatome

- cycles de l'indole

EXEMPLE 19

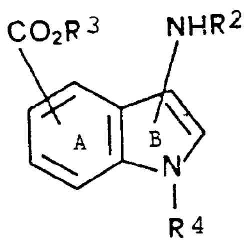

(19)

- cycles du benzofuranne

EXEMPLE 20

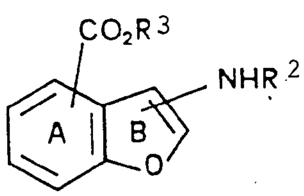

(20)

- cycles du benzothiophène

EXEMPLE 21

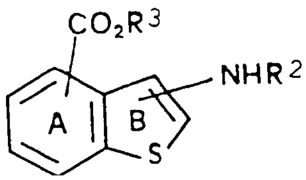

(21)

- cycles du benzimidazole

EXEMPLE 22

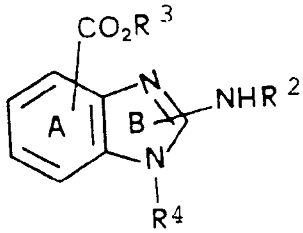

(22)

- cycles des benzothiazoles (primulines l,2 et l,3)

EXEMPLE 23

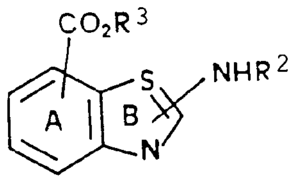

(23)

- cycles de la quinoléine

EXEMPLE 24

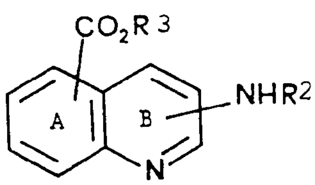

(24)

- cycles de l'isoquinoléine

EXEMPLE 25

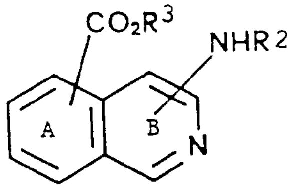

(25)

- cycles des naphthyridines l,8; l,5; l,6

EXEMPLE 26

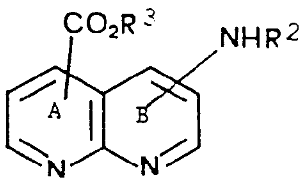

(26)

- cycles des quinozalines

EXEMPLE 27

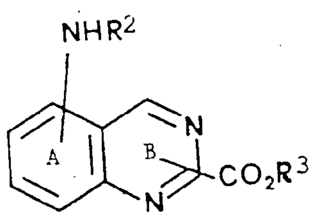

(27)

- cycles de la benzopyridazine 2,3

EXEMPLE 28

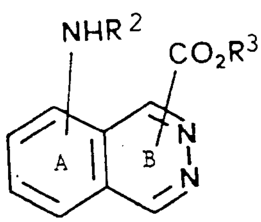

(28)

- cycles de la benzopyridazine l,2

EXEMPLE 29

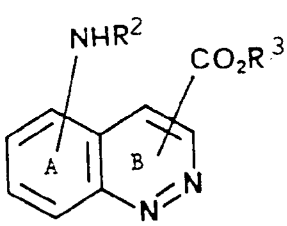

(29)

- cycles de la benzopyrazine

EXEMPLE 30

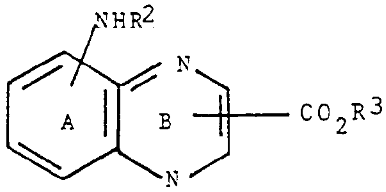

(30)

- cycles des purines (deux hétérocycles)

EXEMPLE 31

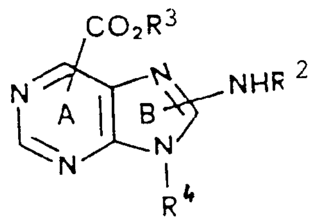

(31)

- cycles des ptéridines (deux hétérocycles)

EXEMPLE 32

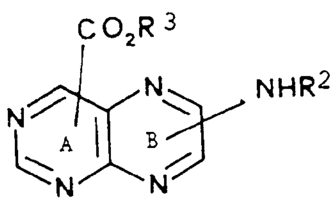

(32)

On notera que, dans les composés des exemples 19 à 32, les groupes $CO_2R^3$ et $NHR^2$ peuvent être soit sur le même cycle A ou B, soit sur deux cycles différents, l'un quelconque sur le cycle A et l'autre sur le cycle B.

c) à trois cycles aromatiques dont au moins un hétérocycle

- cycles des carbazoles.

EXEMPLE 33

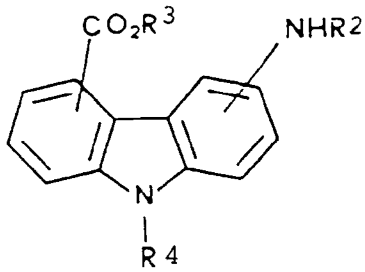

(33)

- cycles des carbolines

EXEMPLE 34

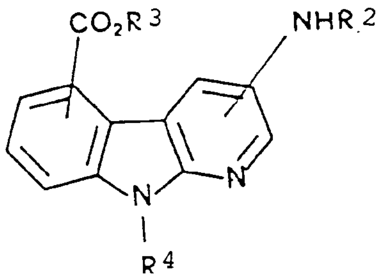

(34)

- cycle du dibenzothiophène

EXEMPLE 35

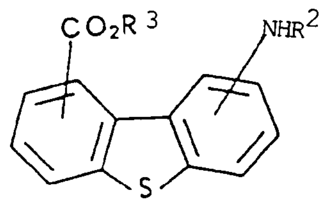

(35)

On notera que pour ces composés également les groupes $CO_2R^3$ et $NHR^2$ peuvent être soit sur le même cycle extrême soit sur deux cycles extrêmes.

II - Exemples d'aldimines issues d'esters d'acides hétérocycliques aminés

EXEMPLE 36

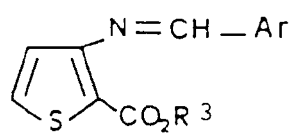

(36)

Ar représentant un groupe aryle

EXEMPLES PLUS GENERAUX

- de type 37

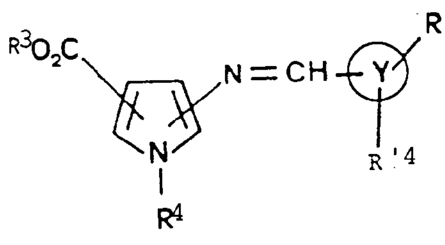

(37)

- de type 38

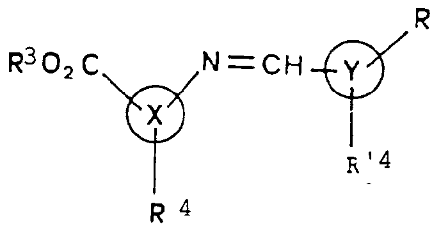

(38)

Dans les formules de types 37 et 38:

– R, $R^3$, $R^4$ et $R'^4$ ont la même définition que dans les formules (II) et (III),

– $R^3O_2C$-X-$R^4$ peut désigner les groupements suivants:

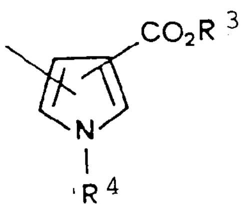

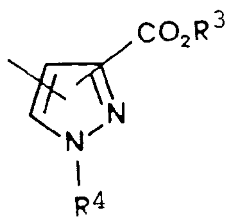

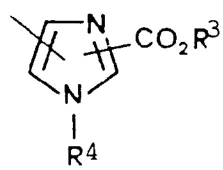

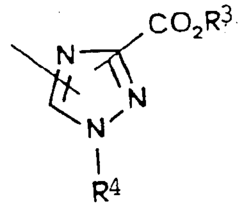

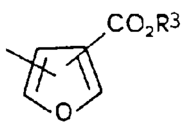
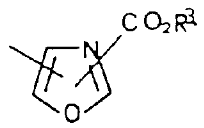
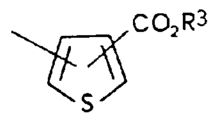
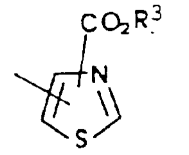

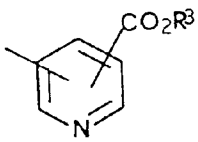
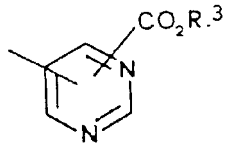
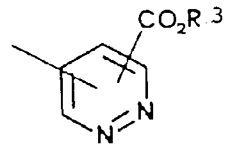
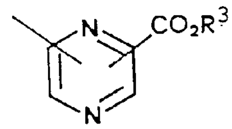

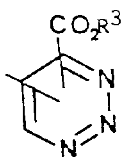
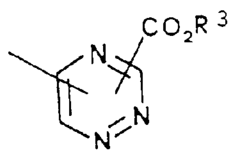
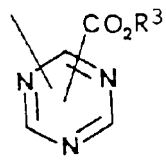

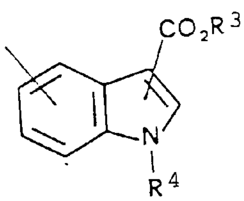
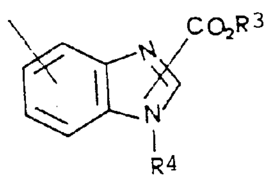
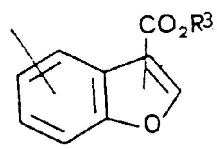
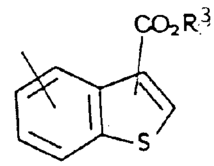

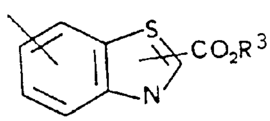
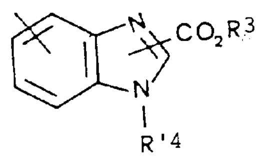
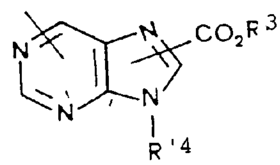

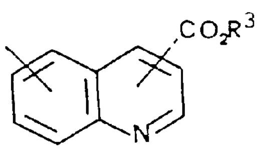
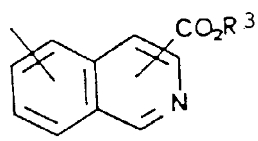

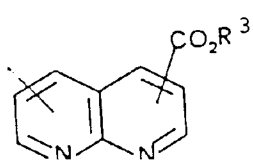
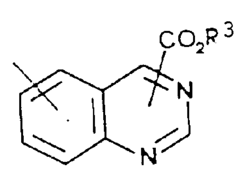
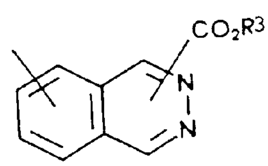

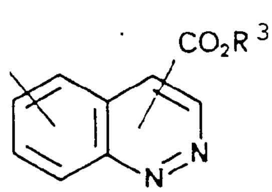
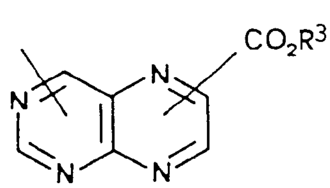

– R-Y-R′4 peut désigner les groupements suivants:

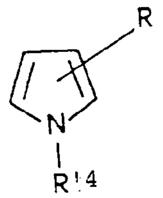
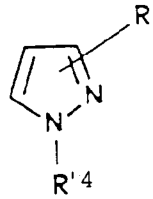
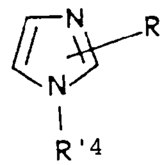
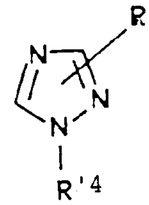

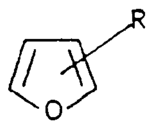
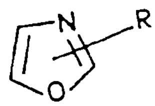
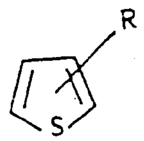
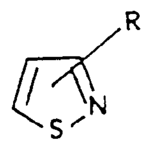

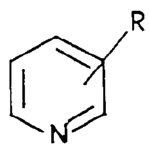
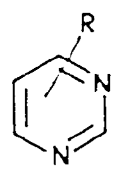
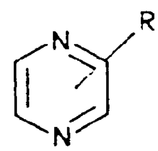
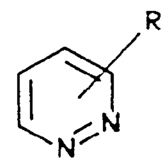

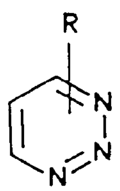
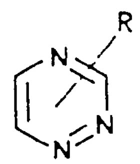
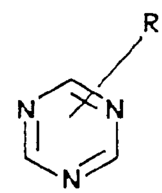

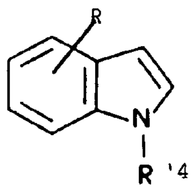
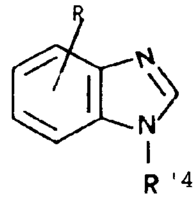
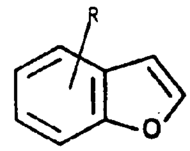
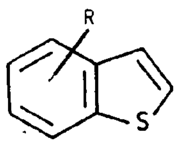
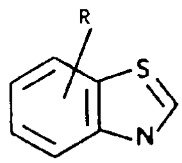
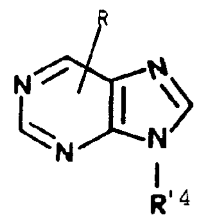
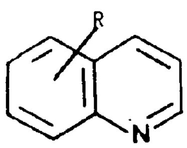
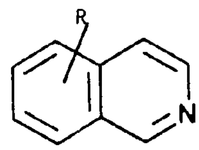
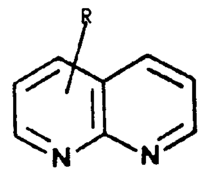
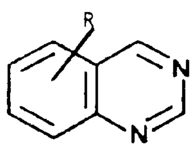
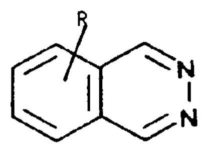
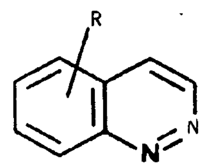
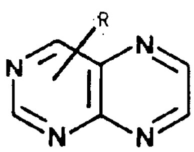

On notera que R peut être placé sur n'importe quel cycle.

B ) EXEMPLES DE PROCEDES DE PREPARATION DES COMPOSES SELON L'INVENTION

Types de réactions pour la synthèse de composés selon l'invention.

On indiquera d'abord les procédés d'obtention d'esters issus de l'acide amino thiophène carboxylique de l'exemple 1, 2 ou 3 de composé et de l'acide amino pyridine carboxylique de l'exemple 4 de composé.

I° - PREPARATION DE COMPOSES THIOPHENIQUES

A - Synthèse de substitution d'un atome de chlore par une fonction amine

I - Le groupement amine peut être crée à partir de la substitution de chlore à l'aide d'ammoniaque en présence de sulfate de cuivre à 175°C sous pression. On peut ainsi à partir du composé (a) obtenir le composé (a')

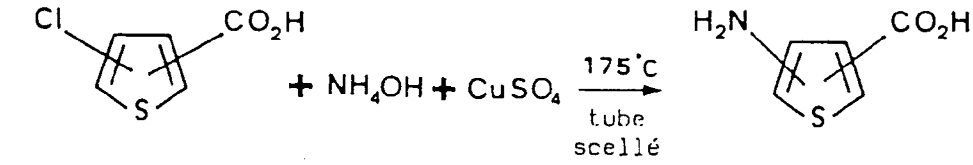


(a) (a')

2 - Le chlorure d'acide (b) peut être estérifié en chloro ester (c).

Celui-ci subit une substitution nucléophile par action de l'amidure de sodium pour fournir l'amino ester de l'exemple 1 (noté 1)

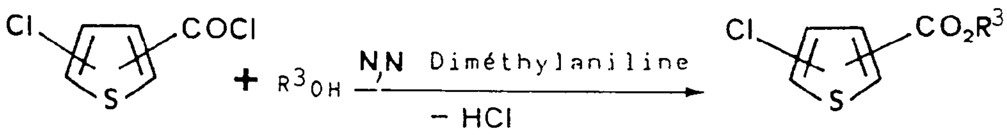


(b) (c)

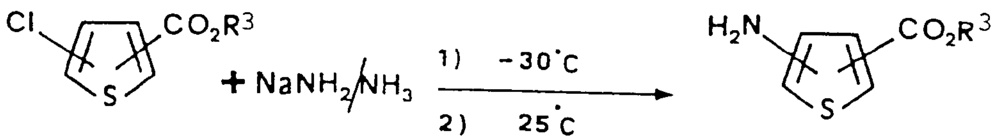


(c) (1)

B - Création du cycle thiophénique

On fait agir le chloro-2 acrylonitrile (d) sur le méthylthioglycolate (e) en présence de méthylate de sodium et on obtient l'amino-3- thiophène-2 carboxylate de méthyle (I')

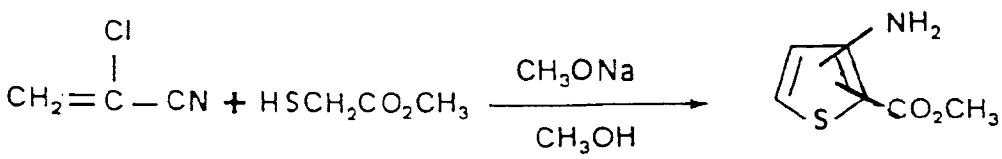


(d) (e) (1')

C - Réactions d'estérification et de transestérification

Ainsi, on peut réaliser la transestérification de l'ester méthylique (I') de l'une des manières ci-après:

– L'alcoolate obtenu en faisant réagir du sodium sur l'alcool (f) est mis à réagir avec le composé de 1' et on obtient l'ester désiré de l'exemple 1 (noté 1)

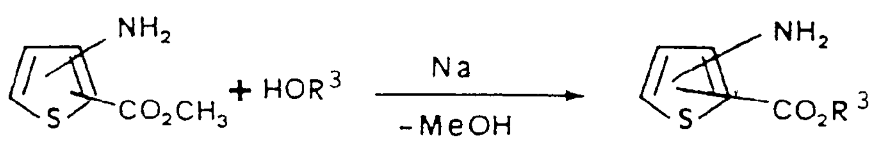


(1') (f) (1)

– Ce dernier composé peut aussi être obtenu en condensant l'ester méthylique (1') avec l'alcool (f) en présence de méthylate de sodium

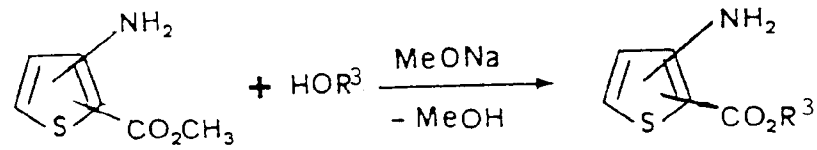


(1') (f) (1)

– L'ester méthylique (1') est mis à réagir avec l'alcool (f) en présence d'un catalyseur métallique de condensation pour obtenir encore (1)

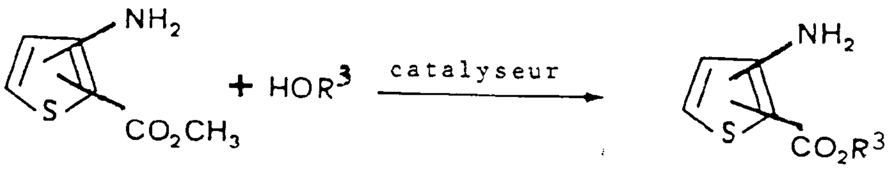


(2) (f) (1)

– L'ester méthylique (1') est transestérifié avec (f) en présence de HCl gazeux ou d'acide paratoluène sulfonique ou d'acide méthanesulfonique et on obtient de nouveau (1).

D - Réactions de substitution sur la fonction amine de (1)

On peut prendre comme exemple la réaction de (1) avec le bromure d'alcoyle $BrR^2$ en présence d'acétate de sodium à reflux et on obtient le composé de l'exemple 2 (noté 2).

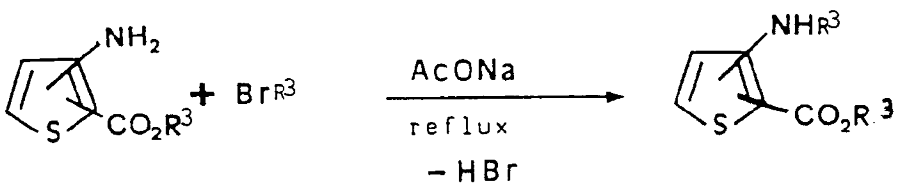


(1) (2)

E - Formation d'aldimines (I") à partir de l'ester (1)

L'ester (1) réagit avec des alcoylaldéhydes ou des arylaldéhydes substitués ou non, pour former des aldimines de type (35), c'est-à-dire des composés selon l'exemple 36.

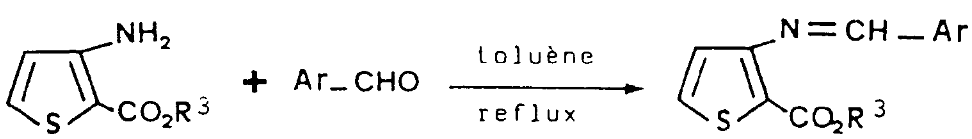


(1) (36)

2° - PREPARATION DE COMPOSES DERIVES DE LA PYRIDINE

A - Synthèses de substitution d'un atome de brome par une onction amine.

On fait réagir un acide bromopyridine carboxylique (g) avec l'ammoniaque en présence du sulfate de cuivre sous pression pour former des composés de type (4)

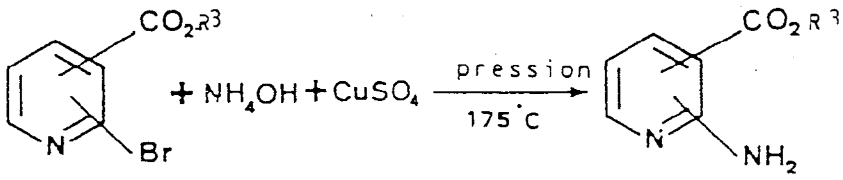


(g) (4)

B - Synthèses réalisées à partir de composés nitrés et halogénés avec substitution d'un atome de chlore par une fonction cyano.

Ainsi on a fait réagir une chloronitro pyridine (h) avec du cyanure cuivreux dans de la DMF à reflux pour préparer (4') en passant par la nitro-cyano pyridine (i)

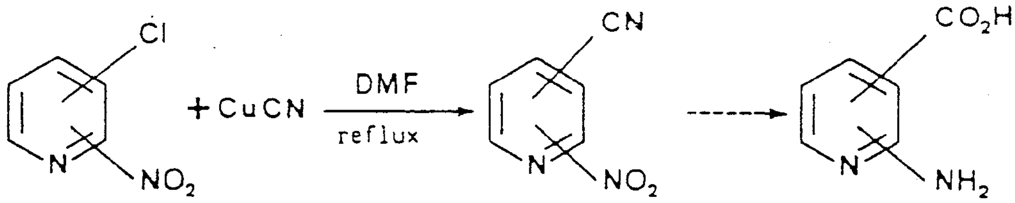


(h) (i) (4')

C - Réduction de nitro-cyano pyridine (i) en amino cyano pyridine (j)

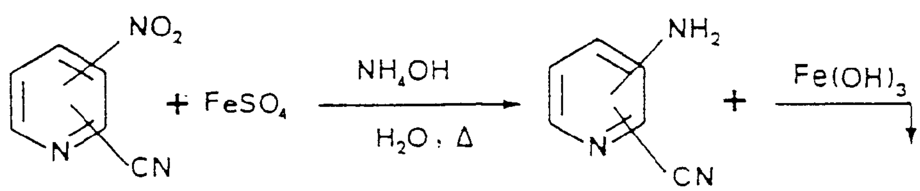


(i) (j)

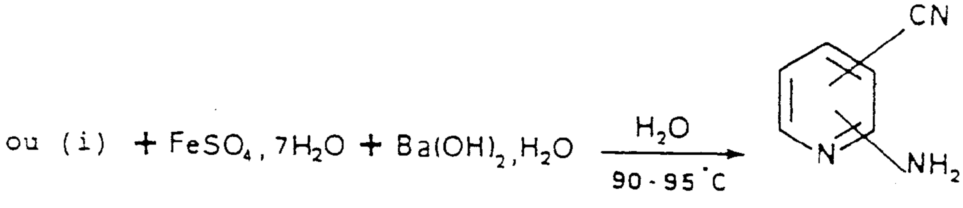


(j)

La nitro-cyano pyridine (i) est réduite dans l'ammoniaque à laquelle on ajoute du sulfate de fer. La réaction

d'oxydo-réduction fournit l'aminocyano pyridine (j).

De même on a réalisé la réduction par la réaction d'hydroxyde ferreux (obtenu in situ par l'action de la baryte sur le sulfate ferreux) sur (i), en obtenant (j).

D - Hydrolyse de l'aminocyano pyridine (j) en acide amino pyridine carboxylique (4).

L'amino cyanopyridine (j), obtenu précédemment, est hydrolysé en acide carboxylique correspondant par l'acide chlorhydrique, ce qui donne l'acide aminopyridine carboxylique (4').

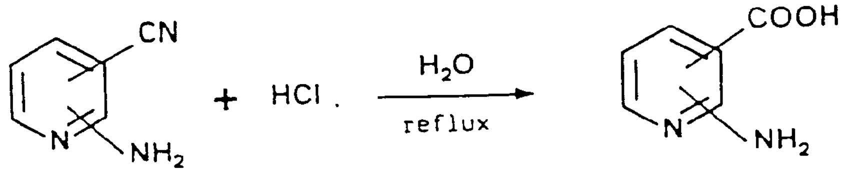


(j) (4')

E - Oxydations menées à partir d'amino-méthyl pyridines:

- par le permanganate de potassium oxydation des amino-méthyl pyridines en ayant pris soin de bloquer la fonction amine avec de l'anhydride acétique.

I - Protection de la fonction amine

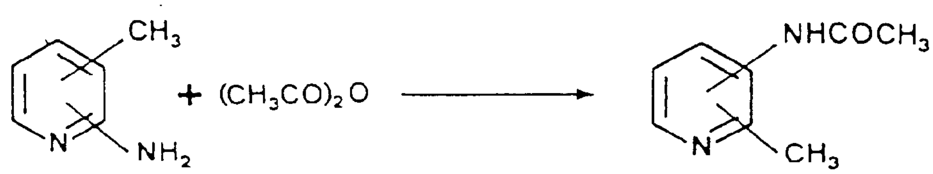


(k) (k')

2 - Oxydation

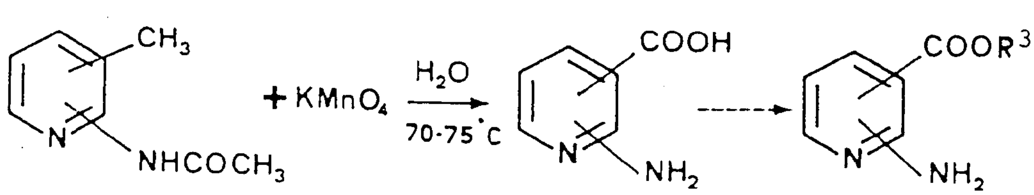


(k) (4') (4)

F - Création du cycle pyridinique par cyclisation

On fait réagir un dérivé du malondialdéhyde (l) sur le chlorhydrate d'éthylamidino acétate (m) en présence de pipéridine et on obtient le produit cyclisé (4").

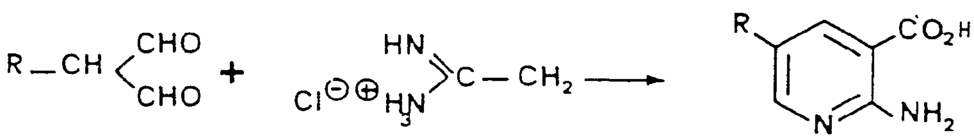

(l) (m) (4")

G - Formation d'acides halogéno pyridine carboxyliques de type (g) (matière première utilisé en A-1 de 2°).

Les acides pyridine carboxylique (o) sont transformés par le chorure de thionyle en chlorures d'acides correspondants (p).

Ceux-ci subissent une bromination à chaud pour fournir des acides bromo pyridine carboxyliques de type (g).

Ils sont transformés en acides amino pyridine carboxyliques (4').

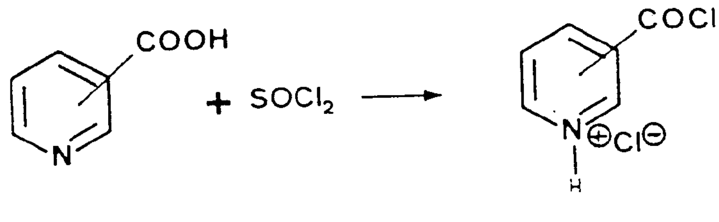


(o) (p)

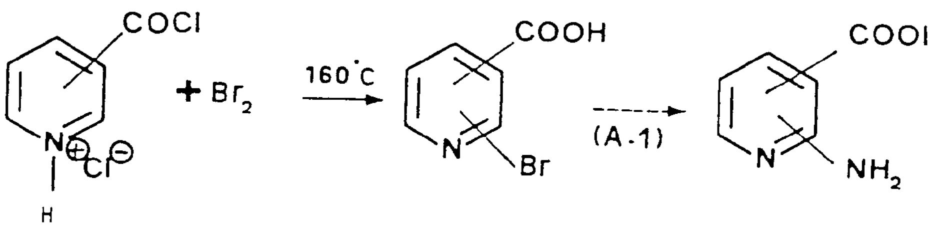


(p) (g') (4')

H - Estérification d'acides aminopyridine carboxyliques de type (4').

L'estérification a été réalisée sur des acides aminés de type (4') soit dans du solvant avec l'alcool adéquat en présence de catalyseur, soit au contact d'acide sulfurique dans du xylène.

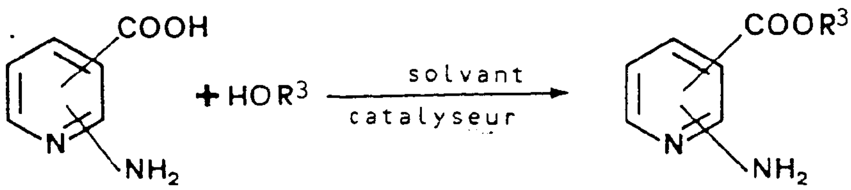


(4) (f) (4)

<u>EXEMPLES PARTICULIERS DETAILLES DE SYNTHESES REALISEES ABOUTISSANT A DES COMPOSES DE TYPE (I)</u>

A - 1. Préparation de composés du type de l'exemple I

On a fait réagir l'acide chloro thiophène carboxylique dans un autoclave (ou un tube scellé) avec de l'ammoniaque en présence de sulfate de cuivre. Le milieu réactionnel est chauffé à 175° pendant 20 heures.

Après refroidissement, on traite avec une solution de sulfite de sodium, puis l'on ajuste le pH de la solution filtrée à pH 5 - 6 afin d'obtenir un précipité qui est filtré et recristallisé dans l'eau.

A - 2. Préparation de composés du type (c)

A un mélange d'alcool de type (f) (l équivalent) et de N, N diméthylaniline (l,l équivalents) porté à reflux dans de l'éther éthylique, on ajoute goutte à goutte le chlorure d'acide (b).

Cette réaction étant exothermique, le chauffage est stoppé durant l'addition.

On porte ensuite à reflux pendant environ 5 heures. Un précipité blanc de chlorhydrate de N,N diméthylaniline se forme sur les parois du récipient.

On ajoute ensuite de l'eau au milieu. On décante. La phase organique est lavée à l'eau, puis avec une solution saturée de bicarbonate de sodium. Après concentration, le résidu est distillé sous pression réduite.

A un large excès d'amidure de sodium dans l'ammoniaque à - 30°C, on ajoute par portions le chlorothénoate alcoyle (c).

Le mélange est agité à - 30°C pendant 4 heures. On ajoute ensuite 1,2 équivalents de chlorure d'ammonium au mélange, puis on laisse revenir la température à l'ambiante.

On extrait à l'éther le composé de l'exemple 1 selon l'invention, que l'on purifie si nécessaire.

B - Création du cycle thiophénique

A une solution de méthylate de sodium dans du méthanol anhydre (2,26 M), on ajoute du méthylthioglycolate (d), puis une solution de chloro - 2 acrilonitrile (e), la température étant maintenue entre 25 et 30°C dans du méthanol (solution 10 M).

On agite le milieu réactionnel pendant environ I heure à la température ambiante. On concentre au l/3 du volume initial. Le résidu est ensuite dilué avec de l'eau, puis extrait à l'éther.

Les phases organiques combinées sont séchées sur $MgSO_4$ et le solvant évaporé.

Le produit (1) est purifié par strippage, puis recristallisation dans un mélange hexane-éther.

C - Réactions d'estérification et de transestérification

C - I. Transestérification de l'ester méthylique (1')

a) On prépare tout d'abord l'alcoolate par réaction du sodium sur l'alcool (f). L'addition de sodium étant terminée, on porte la température à environ 100 - 120 °C (la température dépendant de la longueur de chaîne de l'alcool considéré).

On ajoute ensuite l'ester méthylique (1').

Lorsque la réaction est achevée, on arrête le chauffage. On dilue ensuite avec de l'acétate d'éthyle, on ajoute du noir de carbone, on filtre sur dicalite, évapore le solvant et soumet au strippage le résidu afin d'éliminer l'alcool en excès.

b) L'ester méthylique (l') est ajouté à l'alcool. La température est portée à environ 100 - 120 °C (voir la remarque ci-dessus sous a) jusqu'à obtention d'un milieu homogène, puis on ajoute le catalyseur (acide paratoluène sulfonique).

La température est augmentée jusqu'à élimination du volume théorique de méthanol.

Après refroidissement le mélange réactionnel est traité comme précédemment dans a).

Après mélange des réactifs (1') + (1) avec 2,5 équivalents d'alcool (f), on chauffe jusqu'à obtention d'une solution homogène, puis on ajoute le catalyseur précité (0,2% de la charge totale) et élève la température jusqu'à environ 200°C.

La réaction est arrêtée lorsque le volume d'eau théorique est atteint; l'alcool en excès est éliminé par strippage.

L'ester (1) est purifié comme décrit précédemment.

D - Réactions de substitution sur la fonction amine de (1)

L'ester (1) est ajouté à 3 équivalents de bromure d'alcoyle. Puis on ajoute de l'acétate de sodium (2 équivalents).

Le mélange agité est porté à reflux du bromoalcane pendant environ 48 heures.

Après dilution dans l'éther et filtration, on procède à un lavage à l'eau, puis avec une solution saturée de $NaHCO_3$. La phase éthérée est ensuite séchée sur $MgSO_4$ et le solvant est évaporé.

Le bromoalcane et le N alcoyl ester (2) sont distillés sous pression réduite.

E - Formation d'aldimines à partir de l'ester (1)

A une solution de l'ester (1) dans le toluène (ou le xylène) vers 70-80°C, on ajoute goutte à goutte l'aldéhyde arylique, en léger excès (l,l équivalents).

Le mélange agité sous azote à reflux est chauffé pendant environ 20 heures (jusqu'à ce que le volume d'eau récupéré n'évolue plus).

Le solvant et l'aldéhyde arylique sont distillés sous pression réduite et le produit est purifié.

EXEMPLES PARTICULIERS DETAILLES DE SYNTHESES DE DERIVES DE LA PYRIDINE

A - Substitution d'un atome de brome par une fonction amine:

Réaction avec l'ammoniaque en présence de sulfate de cuivre en tube scellé

Dans un tube en pyrex (ou un réacteur), on mélange de l'acide 5-bromo-nicotinique, de l'ammoniaque (environ 10 équivalents) et du sulfate de cuivre (0,2 équivalent). Puis le tube est scellé et chauffé à 175°C pendant 20 heures. Après refroidissement, le mélange réactionnel est traité par une solution de sulfite de sodium (2 équivalents); il est ensuite filtré et le filtrat amené à pH 4-5; il y a alors formation d'un précipité qui est recristallisé dans l'eau pour aboutir au produit désiré.

B - Synthèses réalisées à partir de composés nitrés et halogénés.

On mélange de la chloronitropyrimidine, du cyanure de cuivre (1,2 équivalents) et du DMF, puis on agite à reflux pendant 4 heures. On verse le mélange réactionnel encore chaud sur une solution d'éthylène diamine (en excès: environ 3 équivalents) dans l'eau. Après agitation, on sépare la phase aqueuse bleue et on l'extrait au benzène; les extraits benzéniques sont ajoutés à la phase organique et lavés avec une solution à 10% de cyanure de sodium et d'eau. Le produit désiré est obtenu après séchage sur du sulfate de magnésium et évaporation du solvant.

C - Réduction de la nitrocyanopyridine

a) La nitrocyanopyridine est dissoute dans un large volume d'eau chaude auquel on ajoute de l'ammoniaque concentrée (environ 15 ml/g de produit). Puis, on ajoute une solution de sulfate de fer (environ 9 équivalents) dans de l'eau chaude, d'un seul trait, dans la solution alcaline et on porte le tout à reflux pendant 30 mn. Le précipité d'hydroxyde de fer est séparé par filtration et la solution refroidie. Une partie du produit précipite alors, le reste précipitant après concentration.

L'amino cyanopyridine brute obtenue peut être recristallisée si désiré.

b) A une suspension d'hydroxyde de fer obtenu à partir de sulfate de fer heptahydraté et d'hydroxyde de barium octahydraté (environ 9 équivalents de chacun d'entre eux), on ajoute, dans un large volume d'eau chaude à 70 - 75°C et sous une forte agitation, de la cyanonitropyridine. La température est alors portée à 90 - 95°C. Après 30 mn de chauffage, on filtre le mélange réactionnel à chaud et on lave le résidu noir à l'eau chaude. La liqueur aqueuse est concentrée sous pression réduite, avec formation d'un précipité qui est filtré; le filtrat est extrait au chloroforme et séché sur $MgSO_4$. Après évaporation du solvant, le solide obtenu est combiné au précipité et recristallisé.

D - Hydrolyse de l'aminocyanopyridine en acide aminopyridine carboxylique

On réalise un mélange d'amino cyanopyridine et d'HCl concentré (37%), puis on porte le tout à reflux pendant environ 2 heures et demie. Après refroidissement, un précipité apparaît; il est filtré. Le reste du produit peut être obtenu en concentrant cette solution. Les solides obtenus sont mélangés et dissous dans un minimum d'eau chaude. Après légère concentration, cette solution est portée au pH 4-5 avec de l'ammoniaque concentrée. On obtient alors un nouveau précipité qui, après refroidissement de la solution, est filtré, puis séché pour conduire à l'acide aminé désiré.

E - Oxydations menées à partir d'aminométhylpyridine

On soumet au reflux pendant 15 à 20 mn l'aminométhylpyridine en présence d'anhydride acétique (2,7 équivalents). L'excès d'anhydride acétique est évaporé sous vide.

L'acétamido obtenu peut être purifié par dilution dans du benzène chaud et reflux pendant 20 mn en présence de noir de carbone. Après filtration, le benzène est évaporé pour donner un produit blanc.

L'acétamido est alors dilué dans de l'eau (jusqu'à une solution de l'ordre de 0,05 M) à 70°C et 2,4 équivalents de permanganate de potassium sont ajoutés d'un seul trait sous une forte agitation.

Le mélange réactionnel est alors agité à 70-75°C pendant 6 heures. Après filtration à chaud sur dicalite, le dioxyde de manganèse est extrait à l'eau chaude; les filtrats sont mélangés et évaporés à sec, puis le solide résiduel est dissous dans un minimum d'eau et la solution acidifiée à pH 3 avec de l'acide chlorhydrique.

La solution acidifiée est ensuite portée à ébullition et ramenée à pH 5 avec de l'ammoniaque concentrée. La solution est laissée toute la nuit à 0°C, ce qui entraîne une précipitation de l'acide amino pyridinique correspondant, qui est filtré, lavé avec une solution d'eau glacée et séché à 110°C.

F - Création du cycle pyridinique par cyclisation

L'hydrochlorure d'éthylamidinoacétate est additionné à une solution d'un malondialdéhyde (l,4 équivalents) dans l'eau. L'addition de quelques gouttes de pipéridine entraîne la précipitation d'un produit qui, recristallisé dans l'acide acétique, conduit au composé attendu.

G - Formation d'acides halogénopyridine carboxyliques

A deux équivalents de chlorure de thionyle, sous atmosphère d'azote, on ajoute l équivalent d'acide nicotinique. Le mélange est ensuite porté à reflux pendant 1/2 heure, puis l'excès de $SOCl_2$ est éliminé sous pression réduite.

Puis on ajoute l équivalent de brome au chlorure d'acide et la température est portée à 160°C pendant 10 heures. On ajoute ensuite 100 ml d'eau glacée au mélange et le pH est amené à 3 par addition de soude. Il se forme alors un précipité, qui est filtré et recristallisé dans de l'éthanol pour conduire à l'acide bromo- 5 nicotinique.

H - Estérification d'acides aminopyridine carboxyliques de type (4').

EXEMPLES DE REACTIONS D'ESTERIFICATION DE COMPOSES PYRIDINIOUES DE TYPE (4')

A. Esters issus de l'acide amino-2 pyridine carboxylique (6)

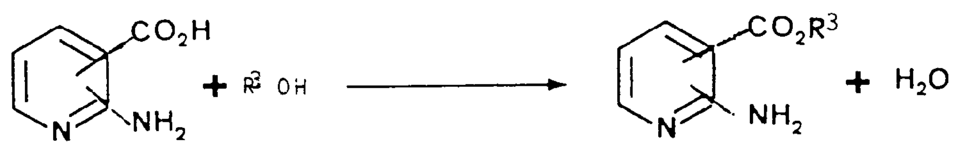

(4') (f) (4)

A un mélange d'acide aminé (4) et d'alcanol (f) en excès (2,5 équivalents) dans du white spirit à environ 100°C, on ajoute le catalyseur (0,28 par rapport à la masse d'acide).

Le milieu est ensuite chauffé à 190-200°C pendant environ 20 heures. On vérifie la fin de la réaction, d'une part, par chromatographie gazeuse, et, d'autre part, par l'observation du volume théorique d'eau recueilli.

Ensuite on filtre à chaud le mélange réactionnel et on distille le filtrat afin d'éliminer l'alcool et le white spirit.

L'ester peut être distillé sous pression réduite, puis recristallisé dans l'éthanol.

A titre d'exemples de composés du type (4) ainsi préparés, on peut citer ceux dans lesquels R est le groupe $C_8H_{17}$; $C_{12}H_{25}$; $C_{16}$ $H_{33}$.

Esters issus de l'acide amino-6 nicotinique (q)

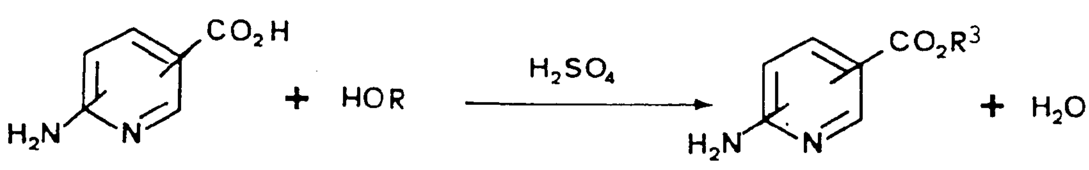


(4') (f) (5')

A un mélange d'acide aminé (4') et d'alcool (f) (en excès: 3 équivalents) porté à 50-60°C, on ajoute goutte à goutte 2, 5 équivalents d'acide sulfurique.

Le mélange est porté à reflux à environ 130-135°C. La fin de la réaction est contrôlée, d'une part, par chromatographie en phase gazeuse et, d'autre part, par l'observation du volume d'eau théorique recueilli. Le temps de réaction est de 20 heures.

A titre d'exemples de composés (5') obtenus par ce procédé, on peut citer ceux dans lesquels R représente

$C_4H9$. $C_6H_{13}$; $C_8H_{17}$.

Dans les tableaux I, II, III et IV ci-après, on indique les résultats de quelques analyses élémentaires effectuées sur des composés préparés comme indiqué ci-dessus.

Esters d'acide amino-3 thiophéne carboxylique 2 Analyses élémentaires

TABLEAU I

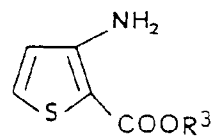

| $R^3$ | C | | H | | N | | S | |
|---|---|---|---|---|---|---|---|---|
| | calc % | Tr % | Calc % | Tr % | Calc % | Tr % | Calc % | Tr % |
| $CH_3$ | 45,85 | 45,95 | 4,49 | 4,42 | 8,91 | 8,84 | 20,40 | 20,52 |
| $C_8H_{17}$ | 61,14 | 60,95 | 8,29 | 8,03 | 5,48 | 5,24 | 12,55 | 12,68 |
| $C_{12}H_{25}$ | 65,55 | 65,29 | 9,38 | 9,46 | 4,50 | 4,64 | 10,29 | 10,33 |

TABLEAU II

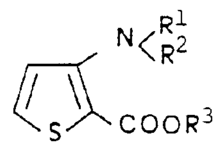

| $R^3$ | $R^1$ | $R^2$ | C | | H | | N | |
|---|---|---|---|---|---|---|---|---|
| | | | Calc % | Tr % | Calc % | Tr % | Calc % | Tr % |
| $C_8H_{17}$ | H | $C_8H_{17}$ | 68,62 | 69,06 | 10,15 | 10,06 | 3,81 | 3,68 |
| $C_8H_{17}$ | $=CH-Ar$ | | 70,14 | 69,60 | 7,33 | 7,56 | 4,09 | 4,26 |
| $C_8H_{17}$ | $=CH$ 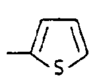 | | 61,86 | 62,35 | 6,63 | 6,90 | 4,01 | 4,32 |

TABLEAU III

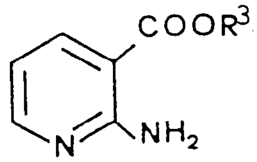

| R3 | C | | H | | N | |
|---|---|---|---|---|---|---|
| | Calc % | Tr % | Calc % | Tr % | Calc % | Tr % |
| $C_8H_{17}$ | 67,17 | 66,96 | 8,86 | 9,04 | 11,19 | 11,14 |
| $C_{12}H_{25}$ | 70,55 | 70,39 | 9,87 | 10,07 | 9,14 | 9,13 |
| $C_{16}H_{33}$ | 72,88 | 72,81 | 10,56 | 10,63 | 7,73 | 7,72 |

TABLEAU IV

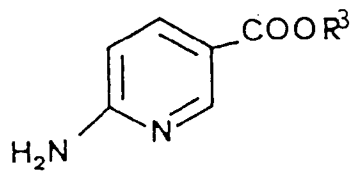

| R3 | C | | H | | N | |
|---|---|---|---|---|---|---|
| | Calc % | Tr % | Calc % | Tr % | Calc % | Tr % |
| $C_4H_7$ | 61,84 | 61,92 | 7,26 | 7,29 | 14,42 | 14,40 |
| $C_6H_{13}$ | 64,84 | 64,90 | 8,16 | 8,21 | 11,19 | 11,15 |
| $C_8H_{17}$ | 67,17 | 67,10 | 8,86 | 8,90 | 11,19 | 11,08 |

C) EXEMPLES DE MISE EN OEUVRE DE COMPOSES SELON L'INVENTION

A titre d'exemple, on indique dans les tableaux A et B ci-après les résultats d'évaluation de tels composés mis en oeuvre en qualité d'anti-oxydants dans une huile de base synthétique de type ester selon les spécifications du projet de norme américaine MIL-L-7808 (K).

Les composés selon l'invention sont ajoutés en une teneur comprise entre 0,l et 10%, de préférence 2,5%

(comme ajouté dans les exemples des tableaux A et B ci-après), aux huiles de graissage du type ester.

On observe que les résultats obtenus, tant sur le plan rhéologique que sur celui de l'oxydation et de la corrosion, sont d'excellents niveaux. On notera la quasi-absence de dépôts et de corrosion sur les éprouvettes des métaux considérés dans le projet.

Deux familles sont citées ici à titre d'exemple:

– les dérivés thiophéniques: Tableau A

– les dérivés pyridiniques: Tableau B

TABLEAU A

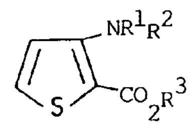

| CATACTERISTIQUES | UNITES | | | | | | LIMITES DU PROJET |
|---|---|---|---|---|---|---|---|
| Aspect à t. ambiante | --- | limpide | limpide | limpide | limpide | limpide | |
| Viscosités à | $mm^2/s$ | | | | | | |
| 100°C | " | 3,98 | 3,99 | 3,99 | 3,98 | 3,97 | min 4,0 |
| 40°C | " | 17,3 | 17,4 | 17,0 | 17,2 | 17,5 | à noter |
| -51°C 35 mn | " | 19598 | 19163 | 17643 | 18173 | 18443 | max 20000 |
| 3 H | " | 19658 | 19369 | 17819 | 18051 | 18439 | " |
| 72 H | " | 19769 | 19308 | 17839 | 18284 | 18822 | " |
| Indice d'acide pH11 | mg KOH/g | 0,12 | 0,19 | 0,27 | 0,09 | 0,11 | max 0,5 |
| Oxydation-corrosion | | | | | | | |
| KV 40°C | % | +22 | +21,5 | +22,9 | +21,0 | +15,5 | max 25 |
| Ia pH 11 | mg KOH/g | +3,2 | +2,25 | +3,20 | +3,7 | 3,2 | max 4 |
| Dépôts | mg/100 ml | 0,5 | 0,8 | +0,65 | 1,0 | 1,25 | |
| Masse Al | $mg/cm^3$ | 0,00 | +0,01 | -0,05 | +0,01 | 0,00 | ± 0,2 |
| Ag | " | +0,05 | -0,06 | -0,13 | -0,06 | +0,02 | ± 0,2 |
| Bronze | " | +0,02 | +0,01 | -0,04 | 0,00 | +0,02 | ± 0,4 |
| Fe | " | +0,04 | +0,02 | 0,00 | +0,02 | +0,02 | ± 0,2 |
| Acier M50 | " | +0,02 | +0,04 | -0,01 | 0,00 | +0,04 | ± 0,2 |
| Mg | " | +0,04 | 0,00 | +0,01 | -0,01 | +0,02 | ± 0,4 |
| Ti | " | +0,02 | -0,01 | 0,00 | | 0,00 | ± 0,2 |
| | | $R^3=C_8H_{17}$ | $R^3=C_{12}H_{25}$ | $R^3=C_8H_{17}$ $R^1=H$ $R^2=C_8H_{17}$ | $R^3=C_8H_{17}$ $R^1=R^2$ : | $R^3=C_8H_{17}$ CH=Ar $R^1,R^2$ = CH- 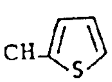 | |

TABLEAU B

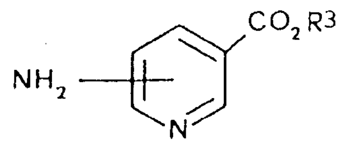

| CARACTERISTIQUES | UNITES | | | | | LIMITES DU PROJET |
|---|---|---|---|---|---|---|
| Aspect à t ambiante | --- | limpide | limpide | | limpide | |
| Viscosités à | $mm^2/s$ | | | | | |
| 100°C | " | 3,98 | 3,97 | 3,97 | 4,00 | min 4,0 |
| 40°C | " | 17,0 | 17,1 | 17,4 | 17,9 | à noter |
| -51°C 35 mn | " | 19128 | 19704 | 19700 | 19100 | max 20000 |
| 3 H | " | 19164 | 19850 | 19900 | 19125 | " |
| 72 H | " | 18855 | 19900 | 20000 | 19580 | " |
| Indice d'acide pH11 | mg KOH/g | 0,25 | 0,32 | 0,22 | 0,26 | max 0,5 |
| Oxydation-corrosion | | | | | | |
| ΔKV 40°C | % | +17,9 | +17,2 | +18,0 | +13,7 | max 25 |
| ΔIa pH 11 | mg KOH/g | +1,46 | + 1,6 | +2,10 | +1,35 | max 4 |
| Dépôts | mg/100 ml | 0,1 | 0,1 | 1 | 1,0 | |
| ΔMasse Al | $mg/cm^3$ | 0,00 | 0,00 | -0,02 | 0,00 | ± 0,2 |
| Ag | " | 0,05 | -0,08 | -0,08 | -0,08 | ± 0,2 |
| Bronze | " | -0,05 | -0,05 | -0,12 | -0,05 | ± 0,4 |
| Fe | " | 0,00 | 0,00 | -0,02 | +0,03 | ± 0,2 |
| Acier M50 | " | +0,01 | +0,02 | +0,02 | +0,03 | ± 0,2 |
| Mg | " | -0,01 | +0,02 | 0,00 | +0,01 | ± 0,4 |
| Ti | " | +0,02 | 0,00 | -0,02 | +0,02 | ± 0,2 |

| | | | | | | |
|---|---|---|---|---|---|---|
| | | $R3=C_8H_{17}$ | $R3=C_{12}H_{25}$ | $R^3=C_{16}H_{33}$ | $R^3=C_8H_{17}$ | |
| | | $-NH_2$ (2) | $-NH_2$ (2) | $-NH_2$ (2) | $-NH_2$ (6) | |

Afin de comparer l'efficacité des produits selon l'invention, on indique dans le Tableau C ci-après les résultats obtenus avec des anti-oxydants (AO) classiques dans la même huile de base de type ester.

Diverses formulations sont données ici en proportions variables d'anti-oxydants. On constate des résultats en net recul par rapport à ceux obtenus avec les anti-oxydants selon l'invention décrits précédemment.

## TABLEAU C

## RESULTATS D'EVALUATION D'ESTERS FORMULES AVEC DES AO CLASSIQUES SELON LES NORMES DU PROJET MIL-L-7808 X

| Composition | Limites Projet 4 cSt<br>$4.10^{-6}$ $m^2/s$ | Ester TMP<br>TAP : 3,0<br>LO1 : 2,0<br>PAN : 2,0<br>BTA : 0,05<br>Quinizarine : 0,02<br>inh. n°1 : 0,15 | Ester TMP<br>TAP : 3,0<br>LO1 : 2,0<br>PAN : 2,0<br>BTA : 0,05<br>Quinizarine : 0,02<br>inh. n° 1 : 0,15 | Ester TMP<br>TAP : 3,0<br>LO1 : 2,0<br>PAN : 2,0<br>BTA : 0,05<br>Quinizarine:0,02<br>Inh. n° 1: 0,25 |
|---|---|---|---|---|
| Aspect | | limpide | limpide | limpide |
| Viscosités à mml/s | | | | |
| 100°C | min 4,00 | 4,01 | 4,05 | 4,00 |
| 40°C | - | 18,2 | 18,2 | 18,2 |
| -51°C 35' | max 30000 | 25500 | 24700 | 26300 |
| 3 h | | 25900 | 24100 | 25700 |
| 72 h | | 26800 | 25500 | 25500 |
| Indice d'acide (mg KOH/g) | max 0,5 | 0,45 | 0,38 | 0,2 |
| Oxydation-corrosion 48 h à 220°C | | | | |
| Δ Ia(mg KOH/g) | max 4,0 | +3,9 | +3,8 | +3 |
| Δ KV 40°C (%) | max 25,0 | +33 | +36 | +35 |
| Dépôts (mg/100ml) | - | 3 | 22 | 3 |
| Δ m(mg/cml) | | | | |
| Al | ± 0,20 | 0 | +0,22 | 40,02 |
| Ag | ± 0,20 | -0,03 | +0,17 | 0 |
| Br | ± 0,40 | 40,03 | +0,21 | 40,10 |
| Fe | ± 0,20 | 40,03 | +0,18 | 40,10 |
| M 50 | ± 0,20 | 40,02 | +0,13 | 40,04 |
| Mg | ± 0,40 | -0,4 | +0,04 | -0,06 |
| Ti | ± 0,20 | 0 | +0,01 | 0 |

TABLEAU C (suite)

RESULTATS D'EVALUATION D'ESTERS FORMULES AVEC DES AO CLASSIQUES SELON LES NORMES DU PROJET MIL-L-7808 X

| Composition | Limites Projet 4 cSt $4.10^{-6}$ $m^2/s$ | Ester TMP<br>TAP : 2,8<br>LO1 : 2,0<br>PAN : 2,0<br>BTA : 0,05<br>Quinizarine : 0,02<br>inh. n°1 : 0,15 | Ester TMP<br>TAP : 3,0<br>LO1 : 2,0<br>Amino 5 Indazole : 0<br>Oxyde de Triphényle phosphine 0,5<br>inh. n° 1 : 0,25 |
|---|---|---|---|
| Aspect | | | limpide |
| Viscosités à mml/s | | | |
| 100°C | min 4,00 | 4,00 | 4,02 |
| 40°C | - | 18,1 | 17,8 |
| -51°C 35' | max 30000 | 25900 | 20500 |
| 3 h | | 26000 | 20700 |
| 72 h | | 26600 | 21000 |
| Indice d'acide (mg KOH/g) | max 0,5 | - | 0,33 |
| Oxydation-corrosion 48 h à 220°C | | | |
| Δ Ia (mg KOH/g) | max 4,0 | +3 | +6,0 |
| Δ KV 40°C (%) | max 25,0 | +30 | +44 |
| Dépôts (mg/100ml) | - | 5 | 14 |
| Δ m(mg/cml) | | | |
| Al | ± 0,20 | -0,04 | 40,02 |
| Ag | ± 0,20 | -0,08 | -0,04 |
| Br | ± 0,40 | -0,05 | -0,02 |
| Fe | ± 0,20 | -0,04 | -0,20 |
| M 50 | ± 0,20 | -0,02 | +0,02 |
| Mg | ± 0,40 | -0,05 | -25 |
| Ti | ± 0,20 | -0,05 | -0,26 |

On va donner maintenant des exemples d'additifs antioxydants et/ou anti-corrosion selon l'invention et leur procédé de préparation, étant entendu que certains des exemples ci-après sont nouveaux, tandis que d'autres décrivent d'un monomère plus détaillé des exemples antérieurs, avec modification de certains détails opératoires et d'essai.

EXEMPLE a

3 - amino picolinate d'octyle

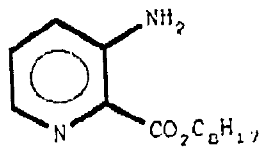

Synthèse en trois étapes

ETAPE I: Cinchoméronimide

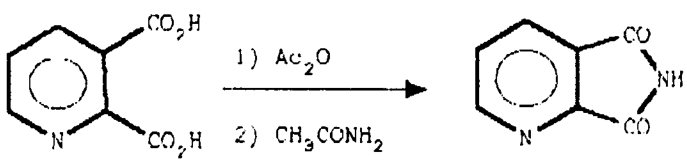


Un mélange de 50g (0,299 mole d'acide cinchoméronique et de 120g (l,75 moles) d'anhydride acétique est porté à l'ébullition sous reflux pendant 10 mn, puis la solution est évaporée sous vide.

50g (0,85 mole) d'acétamide sont ajoutés au résidu. Le milieu est agité et chauffé à reflux pendant 3 heures.

Après un abandon de 24 heures, l'imide cristallise; il est essoré et lavé à l'eau.

PF = 245°C (Rendement 80%)

ETAPE 2: Acide 3-amino picolinique

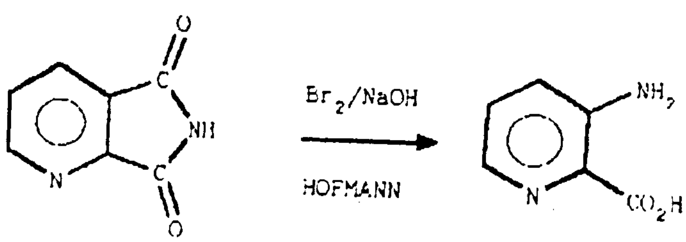


30g (0,202 mole) du cinchoméronimide sont ajoutés à une solution de 33g (0,206 mole) de brome dans 500 ml de soude à 10%.

Le mélange est chauffé à 80-85°C pendant 5 mn, puis refroidi à 40°C et neutralisé à pH 5,25 avec de l'acide acétique.

L'acide aminé cristallise aussitôt; il est recristallisé dans un mélange eau/acide acétique (75/25).

PF > 260°C (Rendement 20%).

ETAPE 3: Amino picolinate d'octyle

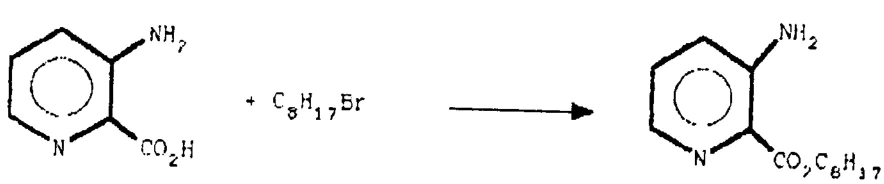

On solubilise 10,5g d'acide aminé dans 100 ml d'une solution éthanolique de potasse à 5,2%.

L'éthanol est ensuite éliminé sous vide et le sel de potassium cristallise.

Celui-ci est mis en suspension dans 150 ml de DMF et 12,2g de l-bromo octane. Ce milieu est chauffé dans un bain d'eau chaude à 80-90°C pendant 6 h.

Le solvant et l'excès de bromooctane sont éliminés par évaporation sous vide. Le résidu est repris par de l'éther et lavé à l'eau carbonatée, puis à l'eau.

La phase organique est séchée sur sulfate de magnésium et évaporée. Le résidu solide est recristallisé dans l'hexane.

PF = 56°C (Rendement 53%)

Infra-rouge: √ ($CO_2$) = 1693 $cm^{-1}$
√ ($NH_2$) = 3429 $cm^{-l}$

RMN ($CDCl_3$): 0,85 (m,3H, $CH_3$)
l,27 (m, 12H, $(CH_2)_6$)
4,28 (t, 2H, $NH_2$)
6,45 (l, 2H, $NH_2$)
6,66 (m, 2H, pyridine)
8,17 (m, lH, pyridine)

Analyse centésimale: $C_{14}H_{22}N_2 0_2$

Calculée: C% = 67,13 H% = 8,79 N% = 11,18

Trouvée: C% = 67,07 H% = 8,98 N% = 11,08

EXEMPLE b

6-amino nicotinate d'hexyle

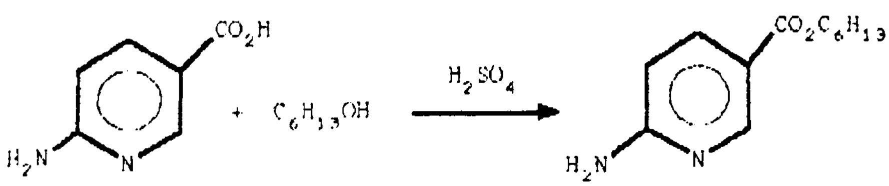


Dans un tricol équipé d'une agitation mécanique et d'un Dean Stark, on introduit 16,2g (0,117 mole) d'acide 6-amino nicotinique dans 35 ml (0,35 mole) de l-hexanol. Le milieu est agité et chauffé à 45°C.

A cette température, on ajoute goutte à goutte 2,5 équivalents (16 ml) d'acide sulfurique concentré.

Après la fin de l'addition, on chauffe à reflux pendant l h supplémentaire. L'excès d'alcool est éliminé par distillation sous vide.

Le résidu est repris par de l'acétate d'éthyle, lavé avec une solution de soude, séché sur $Na_2SO_4$ et évaporé. L'amino nicotinate est recristallisé dans le toluène.

FP = 90°C

Infra-rouge: √ ($CO_2$) = 1694,4 $cm^{-1}$
√ ($NH_2$) = 3415 et 3332 $cm^{-l}$

RMN ($CDCl_3$): 0,85 (m, 3H, $CH_3$)
1,33 (m, 12H, $(CH_2)_6$)
4,28 (t, 2H, 0-$CH_2$)
4,93 (l, 2H, $NH_2$)
4,46 (d, lH, pyridine)
7,96 (d, lH, pyridine)
8,69 (m, lH, pyridine)

Analyse centésimale:

Calculée: C% = 64,84 H% = 8,16 N% = 12,6

Trouvée: C% = 64,69 H% = 8,35 N% = 12,53

EXEMPLE c:

6-amino nicotinate d'octyle

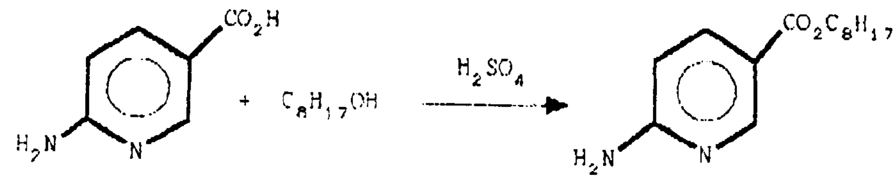


Même mode opératoire que pour l'exemple b.

FP = 90°C

Infra-rouge: √ ($CO_2$) = 3332 et 3416 $cm^{-1}$
√ ($NH_2$) = 1694 $cm^{-l}$

RMN ($CDCl_3$): 0,83 (t, 3H, $CH_3$)
1,1-l,8 (m, 12H, $(CH_2)_6$)
4,26 (t, 2H, $C0_2CH_2$)
5,11 (l, 2H, $NH_2$)
6,45 (d, IH, pyridine)
7,98 (d, IH, pyridine)
8,7 (s, IH, pyridine)

Analyse centésimale:
Calculée: C% = 67,17 H% = 8,86 N% = 11,09
Trouvée: C% = 67,10 H% = 8,90 N% = 11,08

EXEMPLE d

3-amino thiophène 2-dodécyle carboxylate

MODE OPERATOIRE 1

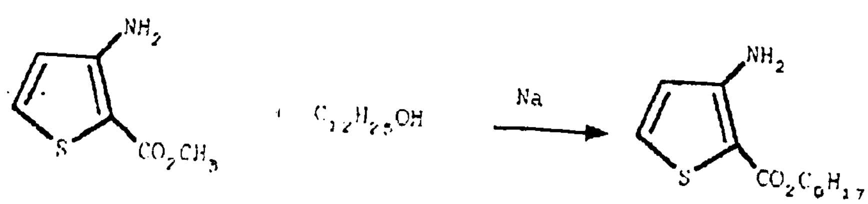


0,2 mole de dodécanol est additionnée à 0,015 mole (7,5%) de sodium.

0,l mole d'amino thénoate de méthyle est additionnée à la solution d'alcoolate et portée à reflux dans un ballon surmonté d'un Dean Stark jusqu'à élimination totale du méthanol. Après dilution dans l'acétate d'éthyle, la solution est lavée à l'eau et séchée sur $MgSO_4$, puis évaporée. Le résidu est distillé sous pression réduite afin d'éliminer l'excès d'alcool. Le solide obtenu est recristallisé dans l'hexane.

FP = 58°C (Rendement = 85%)

Infra-rouge: √ ($CO_2R$) = 1678 $cm^{-1}$
√ ($NH_2$) = 3498 et 3373 $cm^{-l}$

RMN ($CDCl_3$): 0,85 (t, 3H, $CH_3$)
1,2-1,5 (m, 20H, $(CH_2)_6$)
4,19 (t, 2H, $CO_2CH_2$)
5,43 (l, 2H, $NH_2$)
6,53 (d, IH, pyridine)
7,16 (d, IH, pyridine)

Analyse centésimale:
Calculée: C% = 65,55 H% = 9,38 N% = 4,50
Trouvée: C% = 65,53 H% = 9,65 N% = 4,63

MODE OPERATOIRE 2

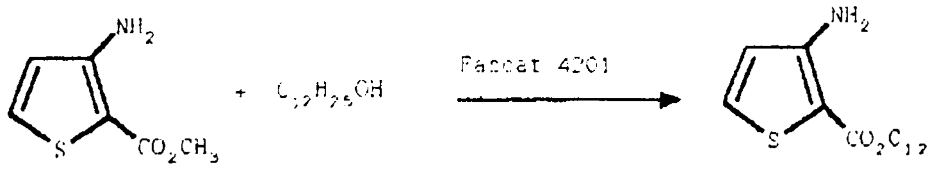


0,I mole d'amino ester et 0,2 mole de dodécanol sont mélangées dans un ballon surmonté d'un Dean Stark.

On ajoute I% de la masse totale de Fascat 4201 (M & T): catalyseur organometallique de transestérification à base d'étain.

Le milieu est chauffé à reflux jusqu'à élimination totale du méthanol. La solution est filtrée sur dicalite et l'alcool excédentaire est éliminé par distillation sous vide. Le résidu est recristallisé dans l'hexane.

EXEMPLE e

Carbamate de dodécyl-3 thiophène

ETAPE 1: Acide thiophène-3 carboxylique

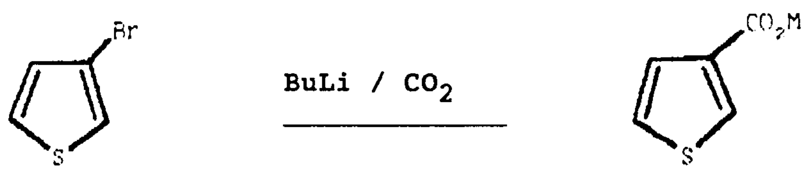


Dans un tricol muni d'un réfrigérant, d'une agitation magnétique, d'une ampoule à brome et d'un thermomètre, on introduit 0,I mole du bromo 3-thiophène, puis 150 ml d'éther anhydre. Le milieu est refroidi à - 70°C sous agitation.

A ce mélange, on ajoute 0,12 mole de butyl lithium (I,65 N) goutte à goutte en maintenant la température à - 70°C; après 4 h de contact à cette température, on fait passer un courant de gaz carbonique pendant 10 mn.

On hydrolyse la solution, puis on décante la phase aqueuse, qui est acidifiée avec de l'acide chlorhydrique dilué à demi. On extrait avec de l'éther éthylique.

Les phases organiques sont lavées à l'eau, séchées sur $MgSO_4$ et concentrées. Le solide blanc obtenu est recristallisé dans le benzène.

L'acide thiophène-2 carboxylique est obtenu avec un rendement de 50%.

PF = 7 6°C.

ETAPE 2: Thiophène-3 carbazide

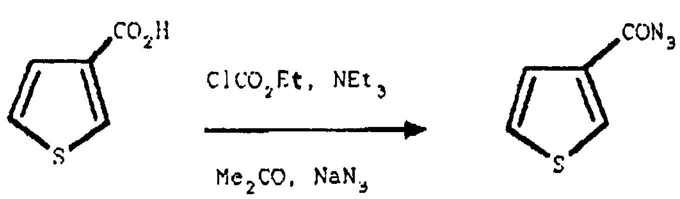


A 0,5 mole d'acide thiophène-3 carboxylique dans 120 ml d'acétone anhydre sous agitation à 0°C est ajoutée 0,6 mole de triéthylamine anhydre.

Après un contact de 15 mn à la même température, on ajoute 0,6 mole de chloroformate d'éthyle dans 10 ml d'acétone.

Après I h de réaction à 0°C, on ajoute 0,6 mole d'azoture de sodium dissous dans un minimum d'eau.

Après I h 30 de réaction, le mélange est versé dans de l'eau glacée. La phase organique est extraite au dichlorométhane et séchée sur $MgSO_4$, puis concentrée à l'évaporateur rotatif.

L'huile obtenue cristallise à froid avec un rendement de 80-85%. Le produit est utilisé sans purification ultérieure.

ETAPE 3: Carbamate de dodécyl-3 thiophène

A 0,5 mole de carboxylazide-3-thiophène dans 120 ml de toluène anhydre est ajoutée 0,75 mole de dodécanol; l'ensemble est porté au reflux durant II h.

Après refroidissement, le solvant est évaporé sous pression réduite; le résidu huileux est repris par de l'éther, puis traité par du charbon animal.

Après filtration, on ajoute le même volume d'éther de pétrole; puis on abandonne le mélange au congélateur durant une nuit. Le précipité formé est collecté par filtration, puis lavé avec de l'éther de pétrole préalablement refroidi.

PF = 82°C (Rendement = 65%)

Infra-rouge: √ $(NH_2)$ = 3260 $cm^{-1}$
√ $(CH_2)$ = 2840 $cm^{-1}$
√ $(CO_2)$ = 1730 $cm^{-1}$

RMN $(CDCl_3)$: 0,8-0,95 (t, 3H, $CH_3$)
1,12-1,5 (m, 18H, $(CH_2)_6$)
1,6-1,8 (t, 2H, $(CH_2)_6$)
4,18 (t, 2H, 0-$CH_2$)
6,85 (s, IH, NH)
6,95 (d, 2H, NH)
7,25 (m, thiophène)

Analyse centésimale:
Calculée: C% = 65,55 H% = 4,50 N% = 9,38
Trouvée: C% = 65,54 H% = 4,52 N% = 9,61

EXEMPLE f

Carbamate d'octyle-3-benzothiophène (Synthèse en 4 étapes)

ETAPE I: Bromo-3-benzothiophène

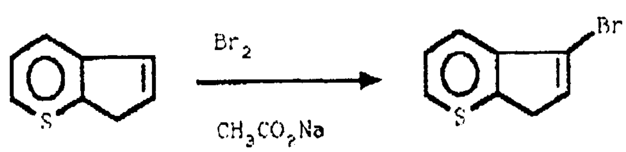


A un mélange de 100g de benzo(b)thiophène et 100g d'acétate de sodium anhydre dans 500 ml de chloroforme anhydre,on ajoute 39 ml de brome goutte à goutte en refroidissant sur un bain de glace.

Après 2 h d'agitation, le mélange est versé dans un mélange d'eau et de glace. Après agitation pendant 30 mn, la phase organique est décantée et lavée plusieurs fois avec de la soude à 5%, puis avec de l'eau et finalement avec une solution saturée de NaCl.

Après séchage sur sulfate de magnésium, filtration et concentration à l'évaporateur rotatif, le résidu huileux est distillé sous pression réduite et le produit est recueilli à 90°C.

ETAPE 2: Acide benzothiophène-3 carboxylique

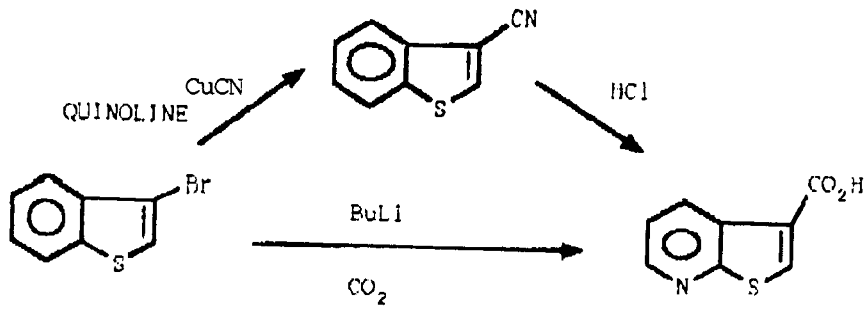


DEUX MODES OPERATOIRES

Mode opératoire a

Dans un tricol de 500 ml, on introduit 50g de bromo-3 thiophène, 300 ml de quinoléine fraîchement distillée, puis 23 g de cyanure cuivreux.

Après 4 h de reflux, le mélange est versé sur de la glace, puis acidifié avec de l'acide chlorhydrique dilué au demi jusqu'à pH=l.

La phase aqueuse obtenue est extraite avec de l'éther; la phase organique est séchée sur $MgSO_4$, puis filtrée et concentrée.

Le résidu huileux obtenu est distillé sous pression réduite; la fraction recueillie est recristallisée dans un mélange eau/alcool.

PF = 66°C (Rendement = 68%)

Calculée: C% = 67,87 H% = 3,86 N% = 8,79

Trouvée : C% = 67,81 H% = 3,15 N% = 8,83

A 0,5 mole de cyano-3 benzo(b)thiophène, on ajoute 50 ml d'acide chlorhydrique concentré; l'ensemble est porté au reflux pendant 2 h.

Après refroidissement, le précipité formé est collecté par filtration, puis recristallisé dans un mélange alcool/eau.

PF = 164°C (Rendement = 45%)

Calculée: C% 60,65 H% = 3,39

Trouvée : C% 60,80 H% = 3,32

Mode opératoire b

A 0,5 mole de bromo-3 benzo(b)thiophène dans 200 ml d'éther sec, on ajoute goutte à goutte 0,6 mole de butyl lithium l,65 N à - 70°C.

Après 4 h de réaction à la même température, un courant de $CO_2$ est mis à barbotter dans la solution pendant 10 mn; la température du milieu s'élève jusqu'à - 10°C; le mélange est hydrolysé par un mélange eau-/glace; la phase aqueuse séparée est lavée avec de l'éther, puis acidifiée par l'acide chlorhydrique dilué au l/2 jusqu'à pH = 2-2,5.

Le précipité formé est collecté par filtration, essoré, puis recristallisé dans un mélange eau/glace.

Le produit est obtenu avec un rendement de 68%.

ETAPE 3: 3-carbazide benzothiophène

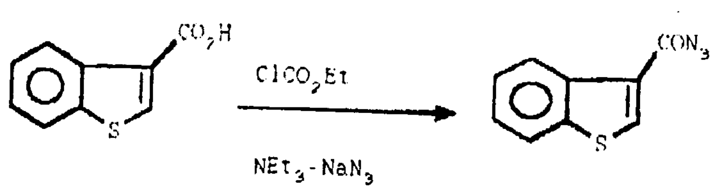


A 0,5 mole d'acide-3 benzo(b)thiophène carboxylique dans 150 ml d'acétone sec sous agitation à 0°C, on ajoute 0,55 mole de triéthylamine anhydre. Après 15 mn de contact à la même température, on ajoute 0,55

mole de chloroformiate d'éthyle dans 15 ml d'acétone sec.

Après l h de réaction à 0°C, 0,55 mole d'azoture de sodium, dissous dans le minimum d'eau, est ajoutée goutte à goutte. Après l h 30 de contact, le mélange est versé dans de l'eau glacée, la phase organique est séparée par addition de dichlorométhane, puis est séchée sur du sulfate de sodium et concentrée à l'évaporateur rotatif. On obtient une huile rougeâtre avec un rendement de 78% qui cristallise à froid; ce composé est utilisé sans aucune purification en raison de sa faible stabilité.

Infra-rouge: √ ($CON_3$) = 2150 $cm^{-1}$

RMN ($CDCl_3$): 7,2-7,65 (m, 2H, benzène)
7,7-8,08 (m, 2H, benzène)
8,7 (s, 1H, thiophène)

ETAPE 4: carbamate d'octyl-3 benzothiophène

A 15 g de carbonylazide-3-benzo(b)thiophène, on ajoute 100 ml de toluène anhydre et 2 équivalents d'alcool octylique; l'ensemble est porté à reflux pendant 16 h.

La solution rougeâtre est traitée par du charbon animal; après filtration et élimination du solvant, le résidu pâteux est solubilisé dans l'éthanol, puis abandonné à -18°C pendant une nuit. Le précipité formé est collecté par filtration, essoré, puis recristallisé dans l'éther de pétrole; le carbamate attendu est isolé avec un rendement de 54%.

PF = 49°C

Infra-rouge: √ (NH) = 3300$cm^{-1}$
√ (CO) = 1700$cm^{-l}$

RMN ($CDCl_3$): 0,75-1,0 (t, 3H, $CH_3$)
1,l-2,0 (m, 12H, $(CH_2)_6$)
4,27 (t, 2H, $0CH_2$)
7,0 (s, 1H, thiophène)
7,2-7,9 (m, 5H, NH + 4H benzène)
6,95 (d, lH, thiophène)
7,25 (m, 2H, thiophène)

Analyse centésimale:

Calculée: C% = 65,55 H% = 4,50 N% = 9,38

Trouvée: C% = 65,54 H% = 4,52 N% = 9,61

EXEMPLE g

N-[thiényl-2- méthylidène]-p-amino octyl benzoate

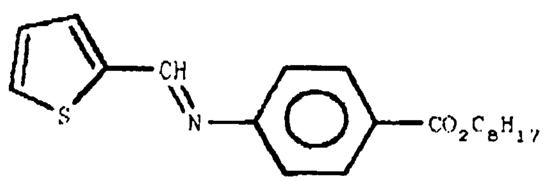

(Synthèse en 2 étapes)

ETAPE l: p-amino benzoate d'octyle

0,l mole d'acide p-amino benzoïque et 0,2 mole d'octanol sont mélangées dans un ballon surmonté d'un Dean Stark. On ajoute dans le milieu l% d'un catalyseur d'estérification organométallique à base d'étain (Fascat 2000 - M & T).

Le milieu est porté au reflux jusqu'à élimination de 0,l mole d'eau.

La solution est alors filtrée sur dicalite et l'excès d'octanol est éliminé par distillation sous vide. L'amino benzoate est recristallisé dans l'hexane.

ETAPE II: N-[thiényl-2 méthylidène]-p-amino octyl benzoate

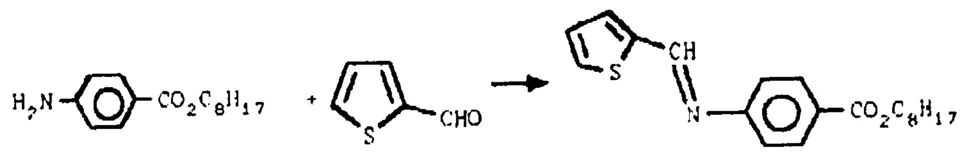

0,l mole d'amino benzoate et 0,1 mole de thiényl-2 carboxaldéhyde sont solubilisées dans du toluène placé dans un ballon muni d'un Dean Stark.

Le milieu est porté au reflux jusqu'à élimination de 0,1 mole d'eau. Le solvant est évaporé sous vide et le résidu est recristallisé dans l'hexane.

Infra-rouge: √ (C =N) = 1620 $cm^{-1}$

RMN ($CDCl_3$): 0,8 (m, 3H, $CH_3$)
1,3 (m, 12H, $CO_2CH_2$)
4,3 (t, 2H, $C0_2CH_2$)
0,5 (m, 1H, CH = N)
7,l-8,l (m, 7H, aromatiques)

Analyse centésimale:

Calculée: C% = 69,93 H% = 7,34 N% = 4,08
Trouvée: C% = 69,73 H% = 7,20 N% = 4,22

EXEMPLE h

N-[thiényl-2 méthylidène]-p-amino dodécyl benzoate

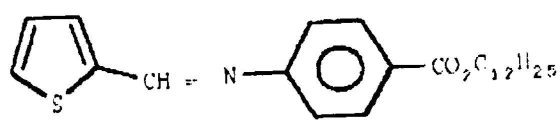

Même mode opératoire que pour l'exemple g, en utilisant 0,2 mole de dodécanol.

Infra-rouge: √ (CH=N) = 1626$cm^{-1}$

Analyse centésimale:

Calculée: C% = 72,14 H% = 8,32 N% = 3,51
Trouvée: C% = 72,28 H% = 8,70 N% = 3,45

EXEMPLE i

N-[thiényl-2 méthylidène]-3-amino 2-octyl thénoate

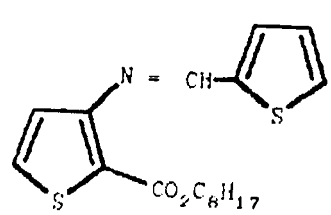

(Synthèse en deux étapes)

ETAPE I: 3-amino 2-octyl thénoate

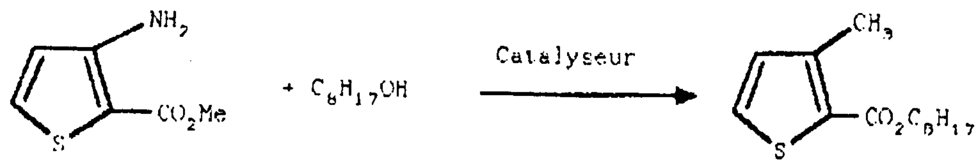


L'ester en $C_8$ est obtenu selon la méthode décrite pour l'exemple d.

ETAPE II: N-[thiényl-2 méthylidène]-3-amino 2-octyl thénoate

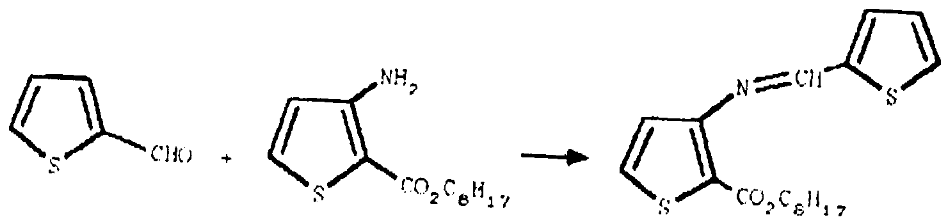

0,l mole de thiophène-2 carboxaldéhyde et 0,l mole de 3-amino thénoate sont solubilisées dans du toluène et placées dans un ballon surmonté d'un Dean Stark. Le milieu est porté à reflux jusqu'à élimination de 0,l mole d'eau. Après évaporation du toluène, le résidu est distillé sous vide.

Eb = 195°C sous 0,5 mmHg

Infra-rouge: √ (C = N) = 1624 $cm^{-1}$
√ ($CO_2$) = 1687 $cm^{-l}$

RMN ($CDCl_3$): 0,81 (t, 3H, $CH_3$)
1,l-l,8 (m, 12H, $CH_2$)
4,24 (t, 2H, $C00CH_2$)
6,24 (d, lH, thiophène: Ha)
6,24 (d, lH, thiophène: Hb)
7,72 (d, 2H, thiophène: Hc)
8,5 (s, lH, CH=N)

Analyse centésimale:

Calculée: C% = 61,86 H% 6,63 N% 4,01
Trouvée : C% = 62,35 H% 7,04 N% 4,32

EXEMPLES DE FORMULATION

Les additifs décrits dans les exemples a à i ont été mis en oeuvre en qualité d'anti-oxydants dans une huile à base synthétique de type ester selon les spécifications du projet de norme américaine MIL-L-7808 (K).

Les composés selon ces exemples sont ajoutés en une teneur comprise entre 0,l et 10%, de préférence l à 3%, aux huiles de graissage de type ester.

On notera que les résultats obtenus (Tableaux A', B', C' ci-après)sont d'un excellent niveau, tant sur le plan rhéologique, que sur celui de l'oxydation et de la corrosion des métaux.

Comme il va de soi, l'invention n'est pas limitée aux modes de réalisation particuliers décrits ni à l'application particulière envisagée. Elle englobe toutes les variantes.

TABLEAU A': Amino-esters hétérocycliques

| CARACTERISTIQUES | UNITES | EXEMPLES a | b | c | d |
|---|---|---|---|---|---|
| Aspect à 0 ambiante | -- | limpide | limpide | limpide | limpide |
| Viscosités à | $mm^2/s$ | | | | |
| 100°C | | 3,86 | 3,98 | 3,98 | 3,99 |
| 40°C | | 16,9 | 17,4 | 17,5 | 17,6 |
| 51°C 35 mn | | 16930 | 19206 | 19432 | 19789 |
| 3 h | | 16920 | 19040 | 19061 | -- |
| 72 h | | 16870 | 19350 | 19082 | 19919 |
| Indice acide pH 11 | mg KOH/g | 0,18 | 0,34 | 0,28 | 0,09 |
| Oxydation-corrosion | | | | | |
| KV 40°C | % | +21,3 | +16,1 | +15,4 | +19,8 |
| Ia pH 11 | mg KOH/g | + 1,7 | + 2,47 | + 2,59 | + 2,8 |
| Dépôts | mg/100ml | + 0,7 | + 1,34 | + 0,26 | + 0,6 |
| Masse Al | $mg/cm^2$ | - 0,02 | - 0,08 | - 0,02 | - 0,02 |
| Ag | | - 0,02 | - 0,10 | - 0,08 | - 0,07 |
| Bronze | | - 0,04 | - 0,14 | - 0,10 | - 0,05 |
| Fe | | 0 | - 0,02 | + 0,02 | + 0,02 |
| Acier | | 0 | - 0,04 | + 0,02 | 0 |
| Mg | | + 0,07 | - 0,06 | 0 | 0 |
| Ti | | 0 | - 0,10 | - 0,02 | + 0,01 |

TABLEAU B': Carbamates

| CARACTERISTIQUES | UNITES | EXEMPLES e | EXEMPLES f |
|---|---|---|---|
| Aspect à 0 ambiante | | limpide | limpide |
| Viscosités à | $mm^2/s$ | | |
| 100°C | | 4,02 | 3,98 |
| 40°C | | 17,5 | 17,5 |
| -51°C 35 mn | | 18585 | 21710 |
| 3 h | | 18100 | 21910 |
| 72 h | | 18830 | 21670 |
| Indice acide pH 11 | mg KOH/g | 0,26 | 0,41 |
| Oxydation corrosion | | | |
| KV 40°C | % | +12,6 | +19,7 |
| Ia pH 11 | mg KOH/g | +13,6 | + 3,15 |
| Dépôts | mg/100ml | +30 | +15,4 |
| Masse Al | $mg/cm^2$ | +0,22 | +0,06 |
| Ag | | +0,18 | +0,02 |
| Bronze | | +0,40 | +0,04 |
| Fe | | +0,21 | +0,10 |
| Acier | | +0,22 | +0,04 |
| Mg | | +0,28 | +0,04 |
| Ti | | +0,18 | +0,06 |

TABLEAU C': Aldimines

| CARACTERISTIQUES | UNITES | EXEMPLES g | h | i |
|---|---|---|---|---|
| Aspect à 0 ambiante | | limpide | limpide | |
| Viscosités à | $mm^2/s$ | | | |
| 100°C | | 4,01 | 3,99 | 3,93 |
| 40°C | | 17,8 | 17,8 | 17,5 |
| -51°C 35 mn | | 19460 | 19716 | 18443 |
| 3 h | | 19580 | 19553 | 18439 |
| 72 h | | 19861 | 19846 | 18822 |
| Indice acide pH 11 | mg KOH/g | 0,14 | 0,11 | 0,11 |
| Oxydation-corrosion | | | | |
| KV 40°C | % | +22,5 | +20,0 | +15,5 |
| Ia pH 11 | mg KOH/g | +3,2 | +3,6 | +3,6 |
| Dépôts | mg/100ml | +2,5 | 0 | +1,25 |
| Masse Al | $mg/cm^2$ | +0,02 | +0,01 | 0 |
| Ag | | -0,03 | +0,01 | +0,02 |
| Bronze | | +0,02 | +0,01 | +0,02 |
| Fe | | +0,04 | +0,01 | +0,02 |
| Acier | | +0,05 | +0,01 | +0,04 |
| Mg | | +0,03 | +0,01 | +0,02 |
| Ti | | +0,03 | +0,01 | 0 |

## Revendications

**1.** Agent anti-oxydant et/ou anti-corrosion, caractérisé en ce qu'il répond à la formule

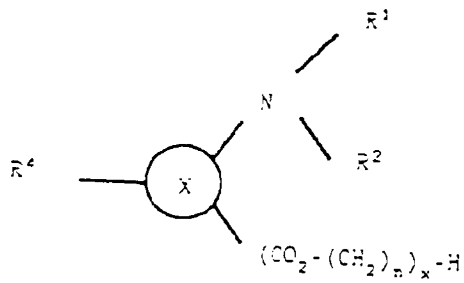

(I)

dans laquelle.

I°) - ⓧ représente un cycle aromatique comportant, ou bien un ensemble de deux ou trois cycles aromatiques comportant chacun:

– 5 ou 6 atomes, dont I à 3 ou I à 4 hétéroatomes, en particulier:

– I à 3 hétéroatomes sur un cycle à 5 atomes ou

– I à 4 hétéroatomes sur un cycle à 6 atomes;

2°) - $R^1$ représente un atome d'hydrogène, ou éventuellement un radical alcoyle inférieur, notamment en $C_6$ à $C_{16}$;

3°) - $R^2$ représente:

a. - un atome d'hydrogène,

b. - un alcoyle en $C_6$-$C_{16}$

c. - un groupe ester $CO_2R^5$, $R^5$ désignant un alcoyle en $C_6$-$C_{16}$,

d. - un groupe $(CH_2)_n$-$CO_2R^6$, $R^6$ désignant un alcoyle en $C_6$ à $C_{16}$ et n représentent un nombre entier entre I et 6,

e. - Ar-$R^7$, Ar désignant un radical aryl et $R^7$ un alcoyle en $C_6$ à $C_{16}$ ou $CO_2R^5$, ou

f - un hétérocycle - $R^7$;

4°) - $R^4$ représente:

| | |
|---|---|
| a. - un atome d'hydrogène | lié: |
| b. - un alcoyl en $C_6$ à $C_{16}$, ou | soit à un atome de C |
| c. - un phényl | soit à un hétéroatome (N); |

5°) - x = O ou I,

– n = un nombre entier de I à 16,

étant entendu que

– si $R^2$ possède un groupement ester (cas c, d, e, f), x = O

– si $R^2$ ne contient pas de groupement ester (cas a, b), x = I

– l'ensemble $R^1$ et $R^2$ peut être constitué par

R

= CH Y (II)

R'4

ce qui correspond à un additif de formule

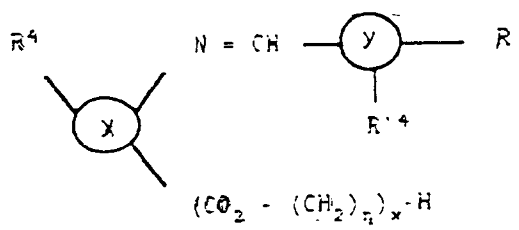

(III)

dans les formules (II) et III:

6°) Ⓨ représente un cycle aromatique (ou sens de Hückel), soit sans hétéroatome (noyau benzénique), soit avec au moins un hétéroatome (N, S ou 0);

7°) - $R'^4$ a la même définition que $R^4$;

8°) - R désigne:

a.- un atome d'hydrogène,

b.- un groupe alcoyle en $C_6$-$C_{16}$,

c.- un groupe ester en $C_6$-$C_{16}$,

étant entendu que

– si R contient un groupement ester (cas c), x = O,

– si R ne contient pas de groupement ester (cas a, b), x = I,

– dans les formules (I), (II) et (III), les différents groupes alcoyles peuvent être ramifiées sur l'un ou plusieurs des atomes de carbone) ou non.

**2.** Application de l'agent selon la revendication I comme additif anti-oxydant et/ou anti-corrosion dans une composition lubrifiante.

**3.** Application de l'agent selon la revendication I comme additif anti-oxydant et/ou anti-corrosion dans une huile lubrifiante destinée à être utilisée à température élevée, telle qu'une huile pour turbine à base ester.

**4.** Composition lubrifiante, caractérisée en ce qu'elle comporte 0.l% à 10% d'agent anti-oxydant et/ou anti-corrosion selon la revendication 1.

**5.** Composition lubrifiante, caractérisée en ce qu'elle comporte 1% à 3% d'agent anti-oxydant ou anti-corrosion selon la revendication I.

Office européen des brevets

# RAPPORT PARTIEL DE RECHERCHE EUROPEENNE

qui selon la règle 45 de la Convention sur le brevet européen est consideré, aux fins de la procédure ultérieure comme le rapport de la recherche européenne

Numero de la demande

EP 91 40 2980

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DE-B-1 055 007 (H. FIESSELMANN) * Revendication; document en entier et particulièrement colonne 2, exemple 1; colonne 3, exemple 1; colonnes 2,4; colonne 4, exemple 5 * --- | 1 | C 07 D 333/38<br>C 07 D 213/80<br>C 10 M 135/34<br>C 10 M 133/40 |
| A | EP-A-0 025 643 (WARNER-LAMBERT CO.) * Revendication 5; page 4, formule IV; page 7, formule VIII; page 11, formule XII; page 55, exemples A et B * --- | 1 | |
| A | DE-A-3 018 134 (BASF AG) * Revendication 8a; page 13, lignes 24-36; page 15, exemple 1 * --- | 1 | |
| A | US-A-4 317 915 (P.N. CONFALONE et al.) * Colonne 1, lignes 15-30; colonne 16, exemple 28; colonne 17, exemple 30; colonne 18, exemple 32; revendications 1-3,5,7,8,12-16,18 * --- -/- | 1 | |
| | | | DOMAINES TECHNIQUES RECHERCHES (Int. Cl.5) |
| | | | C 07 D 333/00<br>C 07 D 213/00<br>C 10 M 133/00<br>C 10 M 135/00 |

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes:

Revendications ayant fait l'objet de recherches incomplètes: 1-5

Revendications n'ayant pas fait l'objet de recherches:

Raison pour la limitation de la recherche:

La définition du radical X dans les revendications est formulée de façon tellement large que la détermination de l'étendue de la protection recherchée n'est pas possible. Les composés existants couverts par certains hétérocycles X revendiqués, sont par ailleurs trop nombreux pour qu'un rapport de recherche exhaustif soit possible. La recherche et le rapport de recherche ont par conséquent été limités aus valeurs de X illustrées par les exemples.

| Lieu de la recherche | Date d'achèvement de la recherche | Examinateur |
|---|---|---|
| LA HAYE | 10-01-1992 | PAISDOR B. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0408)

Office européen des brevets

# RAPPORT PARTIEL DE RECHERCHE EUROPEENNE

Numero de la demande

EP 91 40 2980

| DOCUMENTS CONSIDERES COMME PERTINENTS | | | CLASSEMENT DE LA DEMANDE (Int. Cl. 5) |
|---|---|---|---|
| Catégorie | Citation du document avec indication, en cas de besoin, des parties pertinentes | Revendication concernée | |
| A | EP-A-0 216 290 (BAYER AG)<br>* Colonnes 1-2; colonnes 21,22, exemples 4 -24; revendication 1 *<br>--- | 1 | |
| A | EP-A-0 000 816 (BEECHAM GROUP LTD)<br>* Abrégé; revendication; page 52, exemple 52; page 27, exemples 53-54 *<br>--- | 1 | |
| A | US-A-2 194 567 (R.R. RENSHAW)<br>* Revendication 1; page 1, colonne de droite, exemple 1; page 2, colonne de gauche, exemple 2 *<br>--- | 1 | |
| A | CHEMICAL ABSTRACTS, vol. 97, no. 7, 16 août 1982, page 617, abrégé no. 55495y, Columbus, Ohio, US; & JP-A-82 50 952 (HISAMUTSU PHARMACEUTICAL CO., INC.) 25-03-1982<br>* Abrégé *<br>--- | 1,2 | DOMAINES TECHNIQUES RECHERCHES (Int.Cl. 5) |
| A | US-A-4 187 186 (M. BRAID)<br>* Abrégé; colonne 5, exemple 2; colonne 6, exemple 3, lines 60-68; revendication 1 *<br>----- | 3,4 | |

EPO FORM 1503 03.82 (P0411)